# EUROPEAN PATENT APPLICATION

(11) **EP 4 582 557 A1**
(43) Date of publication of application: **09.07.2025**
(21) Application number: 23860517.4
(22) Date of filing: 31.08.2023
(51) Int. Cl.: C12Q 1/686, C12N 15/09, C12Q 1/6851

(54) **RAPID STERILITY TEST METHOD**

(30) Priority: 01.09.2022 JP 2022139473
(71) Applicant: Shimadzu Diagnostics Corporation, Tokyo 110-8736 (JP)
(72) Inventor: SEKIMATA, Izumi, Yuki-shi, Ibaraki 307-0036 (JP)
(74) Representative: Wächtershäuser & Hartz Patentanwaltspartnerschaft mbB
(86) International application number: PCT/JP2023/031988
(87) International publication number: WO 2024/048755

(57) **Abstract**

An object of the present invention is to provide a rapid sterility test method that reduces inhibition of the nucleic acid amplification reaction of target nucleic acid due to human nucleic acid. The present invention is a rapid sterility test method for detecting presence of bacterial nucleic acid included in a test sample including human nucleic acid by a nucleic acid amplification method, wherein an oligoset that is used in the nucleic acid amplification method comprises (1) one or more probes selected from the group consisting of a probe group PM1, a probe group PM2, and a probe group PM3 and (2) one or more reverse primer in a primer group RM.

## Description

### Technical Field

The present invention relates to a rapid sterility test method for detecting bacterial and/or fungal nucleic acid included in a test sample including human nucleic acid using a nucleic acid amplification method.

### Background Art

In the therapy in the regenerative medicine field, cells taken from a subject are cultured, and the cultured cells may be transplanted into the body of the subject itself or a third person. If the medium, the culture medium after cultivation, or the like that is used in such therapy is contaminated with a microorganism, the patient transplanted with it has a risk of causing infection by the microorganism. Accordingly, it is important to verify sterility of the sample.

As a method for verifying the sterility, both culture and non-culture methods are known. The culture method is a method for detecting microbial growth using a medium or the like in which a target microorganism grows. However, there is a problem of taking time for the microorganism to grow and become detectable. Regarding the non-culture method, polymerase chain reaction (PCR) and real-time PCR are known as the method for detecting nucleic acid of the target microorganism. Non-culture methods can obtain results rapidly compared to culture methods. However, when a sample contains nucleic acid other than that of the target microorganism, there was a problem that the nucleic acid inhibits the nucleic acid amplification reaction of the target microorganism (Non Patent Literature 1).

For these reasons, it has been required to find and provide a rapid sterility test method that is less susceptible to inhibition of the nucleic acid amplification reaction due to human nucleic acid and allows comprehensive microbial detection.

### Citation List

### Non Patent Literature

Non Patent Literature 1: IAN G. WILSON, Inhibition and Facilitation of Nucleic Acid Amplification, APPLIED AND ENVIRONMENTAL MICROBIOLOGY, 63, 3741-3751, (1997)

### Summary of Invention

### Technical Problem

When target nucleic acid included in a test sample contaminated with human-derived cell and/or human nucleic acid is detected by a nucleic acid amplification method, there was a problem that the nucleic acid amplification reaction of the target nucleic acid is inhibited. Accordingly, an object of the present invention is to provide a rapid sterility test method that reduces inhibition of the nucleic acid amplification reaction of target nucleic acid due to human nucleic acid.

### Solution to Problem

Accordingly, the present inventors have conducted various studies in order to solve the above problems and, as a result, quite unexpectedly have found a rapid sterility test method that can reduce inhibition of the nucleic acid amplification reaction of target nucleic acid due to human nucleic acid by using a primer and/or probe comprising a specific nucleotide sequence and have accomplished the present invention.

That is, the present invention provides the following inventions [1] to [19]:
[1] A rapid sterility test method for detecting presence of bacterial nucleic acid included in a test sample including human nucleic acid by a nucleic acid amplification method, wherein an oligoset that is used in the nucleic acid amplification method comprises:
   (1) one or more probes selected from the group consisting of a probe group PM1 of from 12 to 42 nucleotides, including a nucleotide sequence set forth in SEQ ID NO: 18 or a nucleotide sequence having insertion, deletion, or substitution of from 1 to 3 nucleotides in this nucleotide sequence, a probe group PM2 of from 8 to 42 nucleotides, including a nucleotide sequence set forth in SEQ ID NO: 27 or a nucleotide sequence having insertion, deletion, or substitution of from 1 to 3 nucleotides in this nucleotide sequence, and a probe group PM3 of from 7 to 42 nucleotides, including a nucleotide sequence set forth in SEQ ID NO: 30 or a nucleotide sequence having insertion, deletion, or substitution of from 1 to 3 nucleotides in this nucleotide sequence; and
   (2) one or more reverse primers in a primer group RM of from 8 to 30 nucleotides, including a nucleotide sequence set forth in SEQ ID NO: 4 or a nucleotide sequence having insertion, deletion, or substitution of from 1 to 3 nucleotides in this nucleotide sequence.
[2] The rapid sterility test method according to [1], wherein
   the probes are one or more probes selected from the group consisting of nucleotide sequences set forth in SEQ ID NOs: 21 to 26, SEQ ID NO: 29, and SEQ ID NO: 32 or nucleotide sequences having insertion, deletion, or substitution of from 1 to 3 nucleotides in each of these nucleotide sequences; and
   the reverse primers are one or more reverse primers selected from the group consisting of nucleotide sequences set forth in SEQ ID NOs: 7 to 17 or nucleotide sequences having insertion, deletion, or substitution of from 1 to 3 nucleotides in each of these nucleotide sequences.
[3] The rapid sterility test method according to [1] or [2], wherein the oligoset further comprises (3) one or more forward primers in a primer group FM of from 13 to 50 nucleotides, including a nucleotide sequence set forth in SEQ ID NO: 1 or a nucleotide sequence having insertion, deletion, or substitution of from 1 to 3 nucleotides in this nucleotide sequence.
[4] The rapid sterility test method according to [3], wherein the forward primers one or more forward primers selected from the group consisting of a nucleotide sequence set forth in SEQ ID NO: 3 or a nucleotide sequence having insertion, deletion, or substitution of from 1 to 3 nucleotides in this nucleotide sequence.
[5] The rapid sterility test method according to any one of [1] to [4], wherein the test sample includes 1 to 1 × 10⁵ ng of the human nucleic acid.
[6] The rapid sterility test method according to any one of [1] to [5], wherein the number of copies of the bacterial nucleic acid to be detected is 1 to 100 copies.
[7] The rapid sterility test method according to any one of [1] to [6], wherein a nucleic acid extract is used, which is nucleic acid included in a human-derived cell and/or bacterium and extracted from an enzyme-treated sample prepared by adding a nuclease to a test sample at least including the human-derived cell as the human nucleic acid and the bacterium as the bacterial nucleic acid and removing and/or deactivating the nuclease.
[8] The rapid sterility test method according to [7], wherein the number of cells to be detected is 1 to 100 CFU.
[9] A rapid sterility test method for detecting presence of fungal nucleic acid included in a test sample including human nucleic acid by a nucleic acid amplification method, wherein an oligoset that is used in the nucleic acid amplification method comprises:
   (1) one or more forward primers in a primer group FY1 of from 11 to 44 nucleotides, including a nucleotide sequence set forth in SEQ ID NO: 33 or a nucleotide sequence having insertion, deletion, or substitution of from 1 to 3 nucleotides in this nucleotide sequence, and a primer group FY2 of from 16 to 44 nucleotides, including a nucleotide sequence set forth in SEQ ID NO: 45 or a nucleotide sequence having insertion, deletion, or substitution of from 1 to 3 nucleotides in this nucleotide sequence; and
   (2) one or more reverse primers in a primer group RY of from 17 to 44 nucleotides, including a nucleotide sequence set forth in SEQ ID NO: 51 or a nucleotide sequence having insertion, deletion, or substitution of from 1 to 3 nucleotides in this nucleotide sequence.
[10] The rapid sterility test method according to [9], wherein
   the forward primers are one or more forward primers selected from the group consisting of nucleotide sequences set forth in SEQ ID NOs: 37 to 44 or nucleotide sequences set forth in SEQ ID NOs: 46 to 48 or nucleotide sequences having insertion, deletion, or substitution of from 1 to 3 nucleotides in each of these nucleotide sequences; and
   the reverse primers are one or more reverse primers selected from the group consisting of nucleotide sequences set forth in SEQ ID NOs: 52 to 55 or nucleotide sequences having insertion, deletion, or substitution of from 1 to 3 nucleotides in each of these nucleotide sequences.
[11] The rapid sterility test method according to [9] or [10], wherein the oligoset further comprises (3) one or more probes in a probe group PY of from 17 to 50 nucleotides, including a nucleotide sequence set forth in SEQ ID NO: 56 or a nucleotide sequence having insertion, deletion, or substitution of from 1 to 3 nucleotides in this nucleotide sequence.
[12] The rapid sterility test method according to [11], wherein the probes are one or more probes selected from the group consisting of a nucleotide sequence set forth in SEQ ID NO: 58 or a nucleotide sequence having insertion, deletion, or substitution of from 1 to 3 nucleotides in this nucleotide sequence.
[13] The rapid sterility test method according to any one of [9] to [12], wherein the test sample includes 1 to 1 × 10⁵ ng of the human nucleic acid.
[14] The rapid sterility test method according to any one of [9] to [13], wherein the number of copies of the fungal nucleic acid to be detected is 1 to 100 copies.
[15] The rapid sterility test method according to any one of [9] to [14], wherein a nucleic acid extract is used, which is nucleic acid included in a human-derived cell and/or fungus and extracted from an enzyme-treated sample prepared by adding a nuclease to a test sample at least including the human-derived cell as the human nucleic acid and the fungus as the fungal nucleic acid and removing and/or deactivating the nuclease.
[16] The rapid sterility test method according to [15], wherein the number of fungi to be detected is 1 to 100 CFU.
[17] The rapid sterility test method according to any one of [7], [8], [15], and [16], wherein the human-derived cell is in 1 to 1 × 10⁷ cells.
[18] The rapid sterility test method according to any one of [1] to [17], wherein the nucleic acid amplification method is a PCR method or a real-time PCR method.
[19] A real-time PCR kit for use in the rapid sterility test method according to any one of [1] to [18].

### Advantageous Effects of Invention

According to the present invention, it is possible to reduce inhibition of the nucleic acid amplification reaction of target nucleic acid due to human nucleic acid by selecting a primer and/or probe comprising a specific nucleotide sequence. Accordingly, the present invention is significantly excellent as a rapid sterility test method.

### Brief Description of Drawings

Figure 1 shows a calculation equation for determining the copy concentration of a standard DNA solution.
Figure 2 shows amplification curves of nucleic acid amplification reaction using oligoset 1 (human nucleic acid concentration: 1000 ng).
Figure 3 shows amplification curves of nucleic acid amplification reaction using oligoset 2 (human nucleic acid concentration: 1000 ng).
Figure 4 shows amplification curves of nucleic acid amplification reaction using oligoset 2 (human nucleic acid concentration: 100 ng).
Figure 5 shows amplification curves of nucleic acid amplification reaction using oligoset 2 (human nucleic acid concentration: 10 ng).
Figure 6 shows amplification curves of nucleic acid amplification reaction using oligoset 3 (human nucleic acid concentration: 1000 ng).
Figure 7 shows amplification curves of nucleic acid amplification reaction using oligoset 3 (human nucleic acid concentration: 100 ng).
Figure 8 shows amplification curves of nucleic acid amplification reaction using oligoset 3 (human nucleic acid concentration: 10 ng).
Figure 9 shows amplification curves of nucleic acid amplification reaction using oligoset 4 (human nucleic acid concentration: 1000 ng).
Figure 10 shows amplification curves of nucleic acid amplification reaction using oligoset 5 (human nucleic acid concentration: 1000 ng).
Figure 11 shows amplification curves of nucleic acid amplification reaction using oligoset 6 (human nucleic acid concentration: 1000 ng).
Figure 12 shows amplification curves of nucleic acid amplification reaction using oligoset 6 (human nucleic acid concentration: 10 ng).
Figure 13 shows amplification curves of nucleic acid amplification reaction using oligoset 7 (human nucleic acid concentration: 1000 ng).
Figure 14 shows amplification curves of nucleic acid amplification reaction using oligoset 8 (human nucleic acid concentration: 1000 ng).
Figure 15 shows amplification curves of nucleic acid amplification reaction using oligoset 9 (human nucleic acid concentration: 1000 ng).
Figure 16 shows amplification curves of nucleic acid amplification reaction using oligoset 9 (human nucleic acid concentration: 10 ng).
Figure 17 shows amplification curves of nucleic acid amplification reaction using oligoset 10 (human nucleic acid concentration: 1000 ng).
Figure 18 shows amplification curves of nucleic acid amplification reaction using oligoset 11 (human nucleic acid concentration: 1000 ng).
Figure 19 shows amplification curves of nucleic acid amplification reaction using oligoset 12 (human nucleic acid concentration: 1000 ng).
Figure 20 shows amplification curves of nucleic acid amplification reaction using oligoset 13 (human nucleic acid concentration: 1000 ng).
Figure 21 shows amplification curves of nucleic acid amplification reaction using oligoset 13 (human nucleic acid concentration: 100 ng).
Figure 22 shows amplification curves of nucleic acid amplification reaction using oligoset 13 (human nucleic acid concentration: 10 ng).
Figure 23 shows amplification curves of nucleic acid amplification reaction using oligoset 14 (human nucleic acid concentration: 1000 ng).
Figure 24 shows amplification curves of nucleic acid amplification reaction using oligoset 15 (human nucleic acid concentration: 1000 ng).
Figure 25 shows amplification curves of nucleic acid amplification reaction using oligoset 15 (human nucleic acid concentration: 10 ng).
Figure 26 shows amplification curves of nucleic acid amplification reaction using oligoset 16 (human nucleic acid concentration: 1000 ng).
Figure 27 shows amplification curves of nucleic acid amplification reaction using oligoset 17 (human nucleic acid concentration: 1000 ng).
Figure 28 shows amplification curves of nucleic acid amplification reaction using oligoset 18 (human nucleic acid concentration: 1000 ng).
Figure 29 shows amplification curves of nucleic acid amplification reaction using oligoset 19 (human nucleic acid concentration: 1000 ng).
Figure 30 shows amplification curves of nucleic acid amplification reaction using oligoset 20 (human nucleic acid concentration: 1000 ng).
Figure 31 shows amplification curves of nucleic acid amplification reaction using oligoset 21 (human nucleic acid concentration: 1000 ng).
Figure 32 shows amplification curves of nucleic acid amplification reaction using oligoset 21 (human nucleic acid concentration: 10 ng).
Figure 33 shows amplification curves of nucleic acid amplification reaction using oligoset 22 (human nucleic acid concentration: 1000 ng).
Figure 34 shows amplification curves of nucleic acid amplification reaction using oligoset 23 (human nucleic acid concentration: 1000 ng).
Figure 35 shows amplification curves of nucleic acid amplification reaction using oligoset 23 (human nucleic acid concentration: 10 ng).
Figure 36 shows amplification curves of nucleic acid amplification reaction using oligoset 24 (human nucleic acid concentration: 1000 ng).
Figure 37 shows amplification curves of nucleic acid amplification reaction using oligoset 25 (human nucleic acid concentration: 1000 ng).
Figure 38 shows amplification curves of nucleic acid amplification reaction using oligoset 25 (human nucleic acid concentration: 100 ng).
Figure 39 shows amplification curves of nucleic acid amplification reaction using oligoset 25 (human nucleic acid concentration: 10 ng).
Figure 40 shows amplification curves of nucleic acid amplification reaction using oligoset 26 (human nucleic acid concentration: 1000 ng).
Figure 41 shows amplification curves of nucleic acid amplification reaction using oligoset 26 (human nucleic acid concentration: 10 ng).
Figure 42 shows amplification curves of nucleic acid amplification reaction using oligoset 27 (human nucleic acid concentration: 1000 ng).
Figure 43 shows amplification curves of nucleic acid amplification reaction using oligoset 27 (human nucleic acid concentration: 100 ng).
Figure 44 shows amplification curves of nucleic acid amplification reaction using oligoset 27 (human nucleic acid concentration: 10 ng).
Figure 45 shows amplification curves of nucleic acid amplification reaction using oligoset 28 (human nucleic acid concentration: 1000 ng).
Figure 46 shows amplification curves of nucleic acid amplification reaction using oligoset 28 (human nucleic acid concentration: 100 ng).
Figure 47 shows amplification curves of nucleic acid amplification reaction using oligoset 28 (human nucleic acid concentration: 10 ng).
Figure 48 shows amplification curves of nucleic acid amplification reaction using oligoset 29 (human nucleic acid concentration: 1000 ng).
Figure 49 shows amplification curves of nucleic acid amplification reaction using oligoset 30 (human nucleic acid concentration: 1000 ng).
Figure 50 shows amplification curves of nucleic acid amplification reaction using oligoset 30 (human nucleic acid concentration: 10 ng).
Figure 51 shows amplification curves of nucleic acid amplification reaction using oligoset 31 (human nucleic acid concentration: 1000 ng).
Figure 52 shows amplification curves of nucleic acid amplification reaction using oligoset 32 (human nucleic acid concentration: 1000 ng).
Figure 53 shows amplification curves of nucleic acid amplification reaction using oligoset 32 (human nucleic acid concentration: 100 ng).
Figure 54 shows amplification curves of nucleic acid amplification reaction using oligoset 32 (human nucleic acid concentration: 10 ng).
Figure 55 shows amplification curves of nucleic acid amplification reaction using oligoset 33 (human nucleic acid concentration: 1000 ng).
Figure 56 shows amplification curves of nucleic acid amplification reaction using oligoset 33 (human nucleic acid concentration: 100 ng).
Figure 57 shows amplification curves of nucleic acid amplification reaction using oligoset 33 (human nucleic acid concentration: 10 ng).
Figure 58 shows amplification curves of nucleic acid amplification reaction using oligoset 34 (human nucleic acid concentration: 1000 ng).
Figure 59 shows amplification curves of nucleic acid amplification reaction using oligoset 34 (human nucleic acid concentration: 100 ng).
Figure 60 shows amplification curves of nucleic acid amplification reaction using oligoset 34 (human nucleic acid concentration: 10 ng).
Figure 61 shows amplification curves of nucleic acid amplification reaction using oligoset 35 (human nucleic acid concentration: 1000 ng).
Figure 62 shows amplification curves of nucleic acid amplification reaction using oligoset 36 (human nucleic acid concentration: 1000 ng).
Figure 63 shows amplification curves of nucleic acid amplification reaction using oligoset 36 (human nucleic acid concentration: 10 ng).
Figure 64 shows amplification curves of nucleic acid amplification reaction of a bacterium using oligoset 20 (1).
Figure 65 shows amplification curves of nucleic acid amplification reaction of a bacterium using oligoset 20 (2).
Figure 66 shows amplification curves of nucleic acid amplification reaction of a bacterium using oligoset 20 (3).
Figure 67 shows amplification curves of nucleic acid amplification reaction of a bacterium using oligoset 20 (4).
Figure 68 shows amplification curves of nucleic acid amplification reaction of a bacterium using oligoset 20 (5).
Figure 69 shows amplification curves of nucleic acid amplification reaction of a bacterium using oligoset 20 (6).
Figure 70 shows amplification curves of nucleic acid amplification reaction of a bacterium using oligoset 20 (7).
Figure 71 shows amplification curves of nucleic acid amplification reaction of a bacterium using oligoset 20 (8).
Figure 72 shows amplification curves of nucleic acid amplification reaction of a fungus using oligoset 21 (1).
Figure 73 shows amplification curves of nucleic acid amplification reaction of a fungus using oligoset 21 (2).
Figure 74 shows amplification curves of nucleic acid amplification reaction of a fungus using oligoset 21 (3).
Figure 75 shows amplification curves of nucleic acid amplification reaction of a fungus using oligoset 21 (4).

### Description of Embodiments

The present invention is a rapid sterility test method for detecting presence of target nucleic acid included in a test sample including human nucleic acid by a nucleic acid amplification reaction, and which uses an oligoset in the nucleic acid amplification reaction.

The target nucleic acid of the present invention refers to the whole or a part of DNA or RNA of a target microorganism and refers to bacterial nucleic acid when the target microorganism is a bacterium and fungal nucleic acid when the target microorganism is a fungus.

The number of copies in the present invention refers to the total number of regions (nucleic acid amplification regions) as the object of nucleic acid amplification reaction specified by a forward primer and a reverse primer. When nucleic acid has a repeating sequence, a plurality of nucleic acid amplification regions may be present in one genome. For example, when the nucleic acid amplification region of a target microorganism is present in a repeating sequence, if 10 repeating sequences are present in one genome, the number of copies of the nucleic acid is 10. Furthermore, when the 10 genomes are included in a test sample, the number of copies of the target microorganism in the test sample is 100 (10 copies × 10 genomes). In order to guarantee the performance of the rapid sterility test, since it is preferable to be able to detect a small number of copies, the number of copies in a nucleic acid amplification reaction unit is preferably from 1 to 1000, more preferably from 1 to 100, and further more preferably from 1 to 10.

Here, the nucleic acid amplification reaction unit refers to the unit of a nucleic acid amplification reaction. For example, in a real-time PCR method, it means per one reaction well.

As the target nucleic acid, the target microorganism itself may be used. In order to guarantee the performance of the rapid sterility test, it is preferable to be able to detect a small number of the target microorganisms. Accordingly, it is preferable to be able to detect from 1 to 100 colony-forming units (CFU) of target microorganisms in a nucleic acid amplification reaction unit, more preferably from 1 to 50 CFU of target microorganisms, and further more preferably from 1 to 10 CFU of target microorganisms.

The sterility test according to the culture method of The Japanese Pharmacopoeia 18th edition (JP) is required to be able to detect at least 100 CFU of the target microorganism included in the test sample for the sterility test.

The target microorganism is a microorganism that is included or may be included in the test sample. Examples of the microorganism include a target bacterium when the object is a bacterium and a target fungus when the object is a fungus.

In particular, in JP that is a standard method of the sterility test method, examples of the bacterium include Clostridium sporogenes, Pseudomonas aeruginosa, Staphylococcus aureus, and Bacillus subtilis, and examples of the fungus include Aspergillus brasiliensis and Candida albicans, and from the viewpoint of conforming to JP, it is preferable to be able to detect these microorganisms. In addition, also in The EUROPEAN PHARMACOPOEIA 10th edition (EP), the same bacterial species as those in JP are exemplified.

Furthermore, in order to verify whether a test sample is contaminated with microorganisms or not, it is further more preferable to be able to detect microorganisms other than the above microorganisms. Examples thereof include microorganisms separated from indoor environments of product manufacturing room, inspection room, laboratory, and so on (walls, floors, and so on of a building, surfaces of a facility, equipment, and so on, indoor air, raw materials, supplies, water, and so on) and microorganisms that are pathogenic to humans.

The oligoset of the present invention refers to a primer set or a primer and probe set. The primer set is a combination of a forward primer and a reverse primer. The primer and probe set is a combination of a forward primer and a probe, a reverse primer and a probe, or a forward primer, a reverse primer and a probe.

As the forward primer, reverse primer, and probe when a bacterium is a target, the followings can be used.

As a probe for bacteria, a probe group PM can be used. As the probe group PM, one or more probes in a probe group PM1, a probe group PM2, and a probe group PM3 can be used.

The probe group PM1 preferably includes a nucleotide sequence set forth in SEQ ID NO: 18, more preferably includes a nucleotide sequence set forth in SEQ ID NO: 19, further more preferably includes a nucleotide sequence set forth in SEQ ID NO: 20, and even more preferably includes a nucleotide sequence set forth in SEQ ID NO: 21. The nucleotide sequences set forth in these SEQ ID NOs may be nucleotide sequences having insertion, deletion, or substitution of from 1 to 3 nucleotides. The probe group PM1 is preferably of from 12 to 42 nucleotides, more preferably of from 17 to 39 nucleotides, further more preferably of from 20 to 26 nucleotides, and even more preferably of from 23 to 26 nucleotides. Specifically, in the probe group PM1, the probes preferably include any of nucleotide sequences set forth in SEQ ID NOs: 18 to 25 or nucleotide sequences having insertion, deletion, or substitution of from 1 to 3 nucleotides in each of these nucleotide sequences.

The probe group PM2 preferably includes a nucleotide sequence set forth in SEQ ID NO: 27, more preferably includes a nucleotide sequence set forth in SEQ ID NO: 28, and further more preferably includes a nucleotide sequence set forth in SEQ ID NO: 29. The nucleotide sequences set forth in these SEQ ID NOs may be nucleotide sequences having insertion, deletion, or substitution of from 1 to 3 nucleotides. The probe group PM2 is preferably of from 8 to 42 nucleotides, more preferably of from 16 to 39 nucleotides, and further more preferably of from 19 to 22 nucleotides. Specifically, in the probe group PM2, the probes preferably include any of nucleotide sequences set forth in SEQ ID NOs: 23 to 25 and SEQ ID NOs: 27 to 29 or nucleotide sequences having insertion, deletion, or substitution of from 1 to 3 nucleotides in each of these nucleotide sequences.

The probe group PM3 preferably includes a nucleotide sequence set forth in SEQ ID NO: 30, more preferably includes a nucleotide sequence set forth in SEQ ID NO: 31, and further more preferably includes a nucleotide sequence set forth in SEQ ID NO: 32. The nucleotide sequences set forth in these SEQ ID NOs may be nucleotide sequences having insertion, deletion, or substitution of from 1 to 3 nucleotides. The probe group PM3 is preferably of from 7 to 42 nucleotides, more preferably of from 16 to 39 nucleotides, and further more preferably of from 19 to 22 nucleotides. Specifically, in the probe group PM3, the probes preferably include any of nucleotide sequences set forth in SEQ ID NO: 21, SEQ ID NO: 22, SEQ ID NO: 24, SEQ ID NO: 26, and SEQ ID NOs: 30 to 32 or nucleotide sequences having insertion, deletion, or substitution of from 1 to 3 nucleotides in each of these nucleotide sequences.

As a reverse primer for bacteria, a primer group RM can be used.

The primer group RM preferably includes a nucleotide sequence set forth in SEQ ID NO: 4, more preferably includes a nucleotide sequence set forth in SEQ ID NO: 5, further more preferably includes a nucleotide sequence set forth in SEQ ID NO: 6, and even more preferably includes a nucleotide sequence set forth in SEQ ID NO: 7. The nucleotide sequences set forth in these SEQ ID NOs may be nucleotide sequences having insertion, deletion, or substitution of from 1 to 3 nucleotides. The primer group RM is preferably of from 8 to 30 nucleotides, more preferably of from 13 to 27 nucleotides, further more preferably of from 16 to 23 nucleotides, and even more preferably of from 19 to 22 nucleotides. Specifically, in the primer group RM, the primers preferably include any of nucleotide sequences set forth in SEQ ID NOs: 4 to 17 or nucleotide sequences having insertion, deletion, or substitution of from 1 to 3 nucleotides in each of these nucleotide sequences and more preferably include any of nucleotide sequences set forth in SEQ ID NO: 7, SEQ ID NO: 8, and SEQ ID NOs: 10 to 17 or nucleotide sequences having insertion, deletion, or substitution of from 1 to 3 nucleotides in each of these nucleotide sequences.

The combination of the probe group PM and the primer group RM is, from the viewpoint of reducing inhibition of nucleic acid amplification reaction of target nucleic acid due to human nucleic acid, preferably a combination of a nucleotide sequence represented by a probe of from 12 to 42 nucleotides, including a nucleotide sequence set forth in SEQ ID NO: 18 or a nucleotide sequence having insertion, deletion, or substitution of from 1 to 3 nucleotides in this nucleotide sequence, a probe of from 8 to 42 nucleotides, including a nucleotide sequence set forth in SEQ ID NO: 27 or a nucleotide sequence having insertion, deletion, or substitution of from 1 to 3 nucleotides in this nucleotide sequence, or a probe of from 7 to 42 nucleotides, including a nucleotide sequence set forth in SEQ ID NO: 30 or a nucleotide sequence having insertion, deletion, or substitution of from 1 to 3 nucleotides in this nucleotide sequence, and a primer of from 8 to 30 nucleotides, including a nucleotide sequence set forth in SEQ ID NO: 4 or a nucleotide sequence having insertion, deletion, or substitution of from 1 to 3 nucleotides in this nucleotide sequence. The combination is more preferably a combination of a probe including any of nucleotide sequences set forth in SEQ ID NOs: 21 to 26, SEQ ID NO: 29, and SEQ ID NO: 32 or nucleotide sequences having insertion, deletion, or substitution of from 1 to 3 nucleotides in each of these nucleotide sequences and a primer including any of nucleotide sequences set forth in SEQ ID NOs: 7 to 17 or nucleotide sequences having insertion, deletion, or substitution of from 1 to 3 nucleotides in each of these nucleotide sequences, and further more preferably a combination of a probe including a nucleotide sequence set forth in SEQ ID NO: 21 or a nucleotide sequence having insertion, deletion, or substitution of from 1 to 3 nucleotides in this nucleotide sequence and a primer including a nucleotide sequence set forth in SEQ ID NO: 7 or a nucleotide sequence having insertion, deletion, or substitution of from 1 to 3 nucleotides in this nucleotide sequence.

Furthermore, from the viewpoint of comprehensively detecting target bacteria, it is preferable to use a combination of probes including nucleotide sequences set forth in SEQ ID NO: 21 and SEQ ID NO: 22 and primers including nucleotide sequences set forth in SEQ ID NO: 7, SEQ ID NO: 8, and SEQ ID NO: 9. These probes and primers may have insertion, deletion, or substitution of from 1 to 3 nucleotides in each of these nucleotide sequences.

The forward primer for bacteria can be appropriately selected from the group consisting of the following, depending on the target bacterium to be detected. As the forward primer for bacteria, a primer in the primer group FM can be used due to the length of the nucleotide sequence of the reaction product by nucleic acid amplification reaction.

The primer group FM preferably includes a nucleotide sequence set forth in SEQ ID NO: 1, more preferably includes a nucleotide sequence set forth in SEQ ID NO: 2, and further more preferably includes a nucleotide sequence set forth in SEQ ID NO: 3. The nucleotide sequences set forth in these SEQ ID NOs may be nucleotide sequences having insertion, deletion, or substitution of from 1 to 3 nucleotides. The primer group FM is preferably of from 13 to 50 nucleotides, more preferably of from 16 to 40 nucleotides, and further more preferably of from 19 to 22 nucleotides. Specifically, in the primer group FM, the primers preferably include any of nucleotide sequences set forth in SEQ ID NOs: 1 to 3 or nucleotide sequences having insertion, deletion, or substitution of from 1 to 3 nucleotides in each of these nucleotide sequences.

Furthermore, the combination of a forward primer for bacteria and the primer group RM is, from the viewpoint of reducing inhibition of nucleic acid amplification reaction of target nucleic acid due to human nucleic acid, preferably a combination of a primer of from 13 to 50 nucleotides, including a nucleotide sequence set forth in SEQ ID NO: 1 or a nucleotide sequence having insertion, deletion, or substitution of from 1 to 3 nucleotides in this nucleotide sequence, and a primer of from 8 to 30 nucleotides, including a nucleotide sequence set forth in SEQ ID NO: 4 or a nucleotide sequence having insertion, deletion, or substitution of from 1 to 3 nucleotides in this nucleotide sequence. More preferably, it is a combination of a primer including any of nucleotide sequences set forth in SEQ ID NO: 2 and 3 or nucleotide sequences having insertion, deletion, or substitution of from 1 to 3 nucleotides in each of these nucleotide sequences, and a primer including any of nucleotide sequences set forth in SEQ ID NOs: 7 to 17 or nucleotide sequences having insertion, deletion, or substitution of from 1 to 3 nucleotides in each of these nucleotide sequences, and further more preferably, it is a combination of a primer including a nucleotide sequence set forth in SEQ ID NO: 3 or a nucleotide sequence having insertion, deletion, or substitution of from 1 to 3 nucleotides in this nucleotide sequence, and a primer including a nucleotide sequence set forth in SEQ ID NO: 7 or a nucleotide sequence having insertion, deletion, or substitution of from 1 to 3 nucleotides in this nucleotide sequence.

Furthermore, from the viewpoint of comprehensively detecting target bacteria, it is further more preferable to use a combination of a primer set forth in SEQ ID NO: 3 and primers set forth in SEQ ID NO: 7, SEQ ID NO: 8, and SEQ ID NO: 9. These primers may be primers having insertion, deletion, or substitution of from 1 to 3 nucleotides in each of the nucleotide sequences.

Furthermore, the combination of a forward primer for bacteria, the probe group PM, and the primer group RM is, from the viewpoint of reducing inhibition of nucleic acid amplification reaction of target nucleic acid due to human nucleic acid, preferably a combination of a primer of from 13 to 50 nucleotides, including a nucleotide sequence set forth in SEQ ID NO: 1 or a nucleotide sequence having insertion, deletion, or substitution of from 1 to 3 nucleotides in this nucleotide sequence; a probe of from 12 to 42 nucleotides, including a nucleotide sequence set forth in SEQ ID NO: 18 or a nucleotide sequence having insertion, deletion, or substitution of from 1 to 3 nucleotides in this nucleotide sequence, a probe of from 8 to 42 nucleotides, including a nucleotide sequence set forth in SEQ ID NO: 27 or a nucleotide sequence having insertion, deletion, or substitution of from 1 to 3 nucleotides in this nucleotide sequence, or a probe of from 7 to 42 nucleotides, including a nucleotide sequence set forth in SEQ ID NO: 30 or a nucleotide sequence having insertion, deletion, or substitution of from 1 to 3 nucleotides in this nucleotide sequence; and a primer of from 8 to 30 nucleotides, including a nucleotide sequence set forth in SEQ ID NO: 4 or a nucleotide sequence having insertion, deletion, or substitution of from 1 to 3 nucleotides in this nucleotide sequence. More preferably, it is a combination of a primer including any of nucleotide sequences set forth in SEQ ID NO: 2 and 3 or nucleotide sequences having insertion, deletion, or substitution of from 1 to 3 nucleotides in each of these nucleotide sequences, a probe including any of nucleotide sequences set forth in SEQ ID NOs: 21 to 26, SEQ ID NO: 29, and SEQ ID NO: 32 or nucleotide sequences having insertion, deletion, or substitution of from 1 to 3 nucleotides in each of these nucleotide sequences, and a primer including any of nucleotide sequences set forth in SEQ ID NOs: 7 to 17 or nucleotide sequences having insertion, deletion, or substitution of from 1 to 3 nucleotides in each of these nucleotide sequences, and further more preferably, it is a combination of a primer including a nucleotide sequence set forth in SEQ ID NO: 3 or a nucleotide sequence having insertion, deletion, or substitution of from 1 to 3 nucleotides in this nucleotide sequence, a probe including a nucleotide sequence set forth in SEQ ID NO: 21 or a nucleotide sequence having insertion, deletion, or substitution of from 1 to 3 nucleotides in this nucleotide sequence, and a primer including a nucleotide sequence set forth in SEQ ID NO: 7 or a nucleotide sequence having insertion, deletion, or substitution of from 1 to 3 nucleotides in this nucleotide sequence.

Furthermore, from the viewpoint of comprehensively detecting target bacteria, it is preferable to use a combination of a primer set forth in SEQ ID NO: 3, probes set forth in SEQ ID NO: 21 and SEQ ID NO: 22, and primers set forth in SEQ ID NO: 7, SEQ ID NO: 8, and SEQ ID NO: 9. These primers and probes may have insertion, deletion, or substitution of from 1 to 3 nucleotides in each of these nucleotide sequences.

As the forward primer, reverse primer, and probe when a fungus is a target, the followings can be used.

As a forward primer for fungi, a primer group FY can be used. As the primer group FY, one or more primers in a primer group FY1 and a primer group FY2 can be used.

The primer group FY1 preferably includes a nucleotide sequence set forth in SEQ ID NO: 33, more preferably includes a nucleotide sequence set forth in SEQ ID NO: 35, further more preferably includes a nucleotide sequence set forth in SEQ ID NO: 36, and even more preferably includes a nucleotide sequence set forth in SEQ ID NO: 37. The nucleotide sequences set forth in these SEQ ID NOs may be nucleotide sequences having insertion, deletion, or substitution of from 1 to 3 nucleotides. The primer group FY1 is preferably of from 11 to 44 nucleotides, more preferably of from 15 to 41 nucleotides, further more preferably of from 16 to 26 nucleotides, and even more preferably of from 19 to 22 nucleotides. Specifically, in the primer group FY1, the primers preferably include any of nucleotide sequences set forth in SEQ ID NOs: 33 to 48 or nucleotides sequence having insertion, deletion, or substitution of from 1 to 3 nucleotides in each of these nucleotide sequences, and more preferably include any of nucleotide sequences set forth in SEQ ID NOs: 33 to 40, SEQ ID NO: 42, SEQ ID NO: 43, and SEQ ID NOs: 45 to 48 or nucleotide sequences having insertion, deletion, or substitution of from 1 to 3 nucleotides in each of these nucleotide sequences.

The primer group FY2 preferably includes a nucleotide sequence set forth in SEQ ID NO: 45 and more preferably includes a nucleotide sequence set forth in SEQ ID NO: 46. The nucleotide sequences set forth in these SEQ ID NOs may be nucleotide sequences having insertion, deletion, or substitution of from 1 to 3 nucleotides. The primer group FY2 is preferably of from 16 to 44 nucleotides, more preferably of from 19 to 40 nucleotides, further more preferably of from 19 to 37 nucleotides, and even more preferably of from 19 to 22 nucleotides. Specifically, in the primer group FY2, the primers preferably include any of nucleotide sequences set forth in SEQ ID NOs: 45 to 48 or nucleotide sequences having insertion, deletion, or substitution of from 1 to 3 nucleotides in each of these nucleotide sequences.

As a reverse primer for fungi, a primer group RY can be used.

The primer group RY preferably includes a nucleotide sequence set forth in SEQ ID NO: 51 and more preferably includes a nucleotide sequence set forth in SEQ ID NO: 52. The nucleotide sequences set forth in these SEQ ID NOs may be nucleotide sequences having insertion, deletion, or substitution of from 1 to 3 nucleotides. The primer group RY is preferably of from 17 to 44 nucleotides, more preferably of from 18 to 39 nucleotides, further more preferably of from 18 to 36 nucleotides, and even more preferably of from 21 to 24 nucleotides. Specifically, in the primer group RY, the primers preferably include any of nucleotide sequences set forth in SEQ ID NOs: 51 to 55 or nucleotide sequences having insertion, deletion, or substitution of from 1 to 3 nucleotides in each of these nucleotide sequences.

The combination of the primer group FY and the primer group RY is, from the viewpoint of reducing inhibition of nucleic acid amplification reaction of target nucleic acid due to human nucleic acid, preferably a combination of a primer of from 11 to 44 nucleotides, including a nucleotide sequence set forth in SEQ ID NO: 33 or a nucleotide sequence having insertion, deletion, or substitution of from 1 to 3 nucleotides in this nucleotide sequence, or a primer of from 16 to 44 nucleotides, including a nucleotide sequence set forth in SEQ ID NO: 45 or a nucleotide sequence having insertion, deletion, or substitution of from 1 to 3 nucleotides in this nucleotide sequence, and a primer of from 17 to 44 nucleotides, including a nucleotide sequence set forth in SEQ ID NO: 51 or a nucleotide sequence having insertion, deletion, or substitution of from 1 to 3 nucleotides in this nucleotide sequence, more preferably a combination of a primer including any of nucleotide sequences set forth in SEQ ID NOs: 37 to 44 and SEQ ID NOs: 46 to 48 or nucleotide sequences having insertion, deletion, or substitution of from 1 to 3 nucleotides in each of these nucleotide sequences, and a primer including any of nucleotide sequences set forth in SEQ ID NOs: 52 to 55 or nucleotide sequences having insertion, deletion, or substitution of from 1 to 3 nucleotides in each of these nucleotide sequences, and further more preferably a combination of a primer including a nucleotide sequence set forth in SEQ ID NO: 37 or a nucleotide sequence having insertion, deletion, or substitution of from 1 to 3 nucleotides in this nucleotide sequence, and a primer including a nucleotide sequence set forth in SEQ ID NO: 52 or a nucleotide sequence having insertion, deletion, or substitution of from 1 to 3 nucleotides in this nucleotide sequence.

Furthermore, from the viewpoint of comprehensively detecting target fungi, one or more primers in the primer group FY and one or more primers in the primer group RY may be combined.

The probe for fungi can be appropriately selected from the group consisting of the following, depending on the target bacterium to be detected. As the probe for fungi, a probe in the probe group PY can be used.

The probe group PY preferably includes a nucleotide sequence set forth in SEQ ID NO: 56, more preferably includes a nucleotide sequence set forth in SEQ ID NO: 57, and further more preferably includes a nucleotide sequence set forth in SEQ ID NO: 58. The nucleotide sequences set forth in these SEQ ID NOs may be nucleotide sequences having insertion, deletion, or substitution of from 1 to 3 nucleotides. The probe group PY is preferably of from 17 to 50 nucleotides, more preferably of from 20 to 40 nucleotides, and further more preferably of from 23 to 26 nucleotides. Specifically, in the probe group PY, the probes preferably include any of nucleotide sequences set forth in SEQ ID NOs: 56 to 58 or nucleotide sequences having insertion, deletion, or substitution of from 1 to 3 nucleotides in each of these nucleotide sequences.

Furthermore, the combination of the primer group FY, the probe for fungi, and the primer group RM is, from the viewpoint of reducing inhibition of nucleic acid amplification reaction of target nucleic acid due to human nucleic acid, preferably a combination of a primer of from 11 to 44 nucleotides, including a nucleotide sequence set forth in SEQ ID NO: 33 or a nucleotide sequence having insertion, deletion, or substitution of from 1 to 3 nucleotides in this nucleotide sequence, or a primer of from 16 to 44 nucleotides, including a nucleotide sequence set forth in SEQ ID NO: 45 or a nucleotide sequence having insertion, deletion, or substitution of from 1 to 3 nucleotides in this nucleotide sequence; a probe of from 17 to 50 nucleotides, including a nucleotide sequence set forth in SEQ ID NO: 56 or a nucleotide sequence having insertion, deletion, or substitution of from 1 to 3 nucleotides in this nucleotide sequence; and a primer of from 17 to 44 nucleotides, including a nucleotide sequence set forth in SEQ ID NO: 51 or a nucleotide sequence having insertion, deletion, or substitution of from 1 to 3 nucleotides in this nucleotide sequence, more preferably a combination of a primer including any of nucleotide sequences set forth in SEQ ID NOs: 37 to 44 and SEQ ID NOs: 46 to 48 or nucleotide sequences having insertion, deletion, or substitution of from 1 to 3 nucleotides in each of these nucleotide sequences, a probe including any of nucleotide sequences set forth in SEQ ID NO: 57 and 58 or nucleotide sequences having insertion, deletion, or substitution of from 1 to 3 nucleotides in each of these nucleotide sequences, and a primer including any of nucleotide sequences set forth in SEQ ID NOs: 52 to 55 or nucleotide sequences having insertion, deletion, or substitution of from 1 to 3 nucleotides in each of these nucleotide sequences, and further more preferably a combination of a primer including a nucleotide sequence set forth in SEQ ID NO: 37 or a nucleotide sequence having insertion, deletion, or substitution of from 1 to 3 nucleotides in this nucleotide sequence, a probe including a nucleotide sequence set forth in SEQ ID NO: 58 or a nucleotide sequence having insertion, deletion, or substitution of from 1 to 3 nucleotides in this nucleotide sequence, and a primer including a nucleotide sequence set forth in SEQ ID NO: 52 or a nucleotide sequence having insertion, deletion, or substitution of from 1 to 3 nucleotides in this nucleotide sequence.

Furthermore, from the viewpoint of comprehensively detecting target fungi, one or more primers in the primer group FY, one or more probes for fungi, and one or more primers in the primer group RM may be combined.

The human nucleic acid of the present invention refers to nucleic acid of a human-derived cell. Examples of the human-derived cell include, but not limited to, cells taken from a human body and cells obtained by modifying the cells (including, but not limited to, cells that have been treated, such as an established cell line, iPS cell, and ES cell) and cells obtained by culturing these cells.

A concentration of human nucleic acid can be measured by a known method. For example, the nucleic acid of human-derived cells is extracted, and the concentration thereof can be measured using a micro-volume spectrophotometer NanoDrop (Thermo Fisher Scientific K.K.). The concentration of human nucleic acid is, from the viewpoint of reducing inhibition of nucleic acid amplification reaction of target nucleic acid due to human nucleic acid, preferably from 1 to 1 × 10⁵ ng in a nucleic acid amplification reaction unit, more preferably from 10 to 1 × 10⁴ ng, further more preferably from 100 to 5000 ng, and even more preferably from 1000 to 2500 ng. The proportions of target nucleic acid to human nucleic acid is, from the viewpoint of reducing inhibition of nucleic acid amplification reaction of target nucleic acid due to human nucleic acid, preferably from 10 to 1000 ng of human nucleic acid relative to 1000 copies of target nucleic acid, more preferably from 10 to 1000 ng of human nucleic acid relative to 100 copies of target nucleic acid, and further more preferably from 10 to 1000 ng of human nucleic acid relative to 10 copies of target nucleic acid.

As the human nucleic acid, human-derived cells themselves can also be used. The number of human-derived cells is, from the viewpoint of reducing inhibition of nucleic acid amplification reaction of target nucleic acid due to human nucleic acid, preferably from 1 to 1 × 10⁷ cells in a nucleic acid amplification reaction unit, more preferably from 1000 to 1 × 10⁶ cells, further more preferably from 1 × 10⁴ to 5 × 10⁵ cells, and even more preferably from 1 × 10⁵ to 2.5 × 10⁵ cells.

The test sample of the present invention refers to a specimen that becomes an object of the sterility test, and examples thereof include water, a buffer, physiological saline, a biological sample (such as blood, urine, saliva, and cerebrospinal fluid), a cell culture medium, a culture supernatant, and a medium. It is desirable to include human-derived cells and/or human nucleic acid. Although the test sample can also be used directly, in order to efficiently collect a target microorganism in a test sample, a specimen containing collected microorganisms (microbial specimen) may be used, and in order to increase the detection sensitivity of the rapid sterility test, a target nucleic acid extract-containing specimen (nucleic acid extraction specimen) may be used. When a test sample at least includes human-derived cells and a target microorganism, a specimen after nuclease treatment and then nucleic acid extraction (enzyme-treated specimen) can be used.

The rapid sterility test method of the present invention is a method for detecting target nucleic acid including in a test sample, using a nucleic acid amplification method or the like. When target nucleic acid is not detected by the rapid sterility test method, it is possible to judge that the target microorganism is not present in the test sample subjected to the test.

The nucleic acid amplification method of the present invention may be a known method, and examples thereof include a PCR method and a real-time PCR method. In particular, from the viewpoint of being able to verify the amplification status in real time, the real-time PCR method is preferable. Examples of the real-time PCR method include an intercalator method and a probe method. As the intercalator, for example, cyber green is known. As the probe method, for example, a TaqMan probe method is known, and from the viewpoint of the specificity to target nucleic acid, the TaqMan probe method is preferable.

As the reaction conditions of the nucleic acid amplification method, usual thermal denaturation, hybridization, and elongation may be performed, and detailed conditions can be appropriately changed depending on the enzyme and oligoset to be used.

As a method for producing a microbial specimen, for example, a method for collecting bacteria by adding a protein and/or a hydrophobic carrier to a test sample, more specifically, a method described in PCT/JP/2018/016676 and PCT/JP/2019/41658 may be used. Briefly, a protein such as BSA is added alone or in addition to a hydrophobic carrier such as latex particles to a microbial specimen, and followed by centrifugation to collect the precipitate to collect bacteria. Consequently, the target microorganism can be efficiently collected.

The method for producing a nucleic acid extraction specimen may be a known method. The nucleic acid extraction is divided roughly into two steps of bacteriolysis and purification.

Examples of the method for bacteriolysis include, but not limited to, physical crushing such as bead crushing and enzymatic treatment using an enzyme that bacteriolyses each bacterium. From the viewpoint of being capable of crushing bacteria without depending on bacterial species, physical crushing is preferable.

Examples of the purification include, but not limited to, a magnetic bead method, a spin-column method, and a phenol-chloroform method.

The materials that are used for bacteriolysis and purification are preferably those not containing nucleic acid in the materials and further are preferably those easy to handle. For example, as a method for purification, QIAampUCP DNA Micro kit adopting a spin-column method is exemplified.

In a method for producing an enzyme-treated specimen, the specimen can be obtained by adding a nuclease to a test sample at least including human-derived cells and a target microorganism, removing and/or deactivating the nuclease, and then performing nucleic acid extraction. The nuclease cannot degrade the nucleic acid present in a cell, but can degrade free nucleic acid. The free nucleic acid is extracellular nucleic acid, and examples thereof include, but not limited to, nucleic acid leaked outside the cell. Consequently, only the target microorganism having proliferation potential can be detected by the nucleic acid amplification method.

The nuclease is not particularly specified, but TurboNuclease is preferable. The method for extracting nucleic acid used for the enzyme-treated specimen is not particularly specified, and may be a known method.

The present invention may be formed into a nucleic acid amplification kit including an oligoset. Examples of the nucleic acid amplification kit include, but not limited to, a PCR kit and a real-time PCR kit. Specifically, in a rapid sterility test for bacteria, the nucleic acid amplification kit includes the above primer group FM and primer group RM, or the primer group FM, primer group RM, and probe group PM, and in a rapid sterility test for fungi, the nucleic acid amplification kit includes the above primer group FY and primer group RY, or the primer group FY, primer group RY, and probe group PY.

The kit may include a bacterium-collecting kit, a reagent for destroying cells other than the target microorganism, a nucleic acid extraction kit, a reagent for degrading free nucleic acid, a protocol for performing the test, and so on. The kit can be used as a rapid sterility test for rapidly detecting target nucleic acid included in a test sample.

### Examples

The present invention will now be described in more detail with reference to Examples, but the present invention is not limited in any way by these Examples.

### Example 1

### [Oligoset]

Oligosets used in Examples were shown in Table 1.

The primers and probes used in Examples were designed using sequence databases of bacteria and fungi. The primers and probes are targeted to regions encoding 16S ribosomal RNA in bacteria and 18S ribosomal RNA in fungi, respectively.

Oligosets that combined the designed primers and probes were compared with the regions encoding 16S or 18S ribosomal RNA of a microorganism as the object to verify whether all of the forward primer, probe, and reverse primer in each combination can be 100% identical to the regions or not. For example, when parts of the sequence of a bacterial strain in a certain bacterial species is 100% identical to the sequences of SEQ ID NO: 3, SEQ ID NO: 7, and SEQ ID NO: 21, respectively, regarding the bacterial species, all of the forward primer, probe, and reverse primer were judged to be a combination that can be 100% identical and to be able to detect the bacterial species.

As an example, the results when oligoset 20 and oligoset 21 were used for bacteria and fungi, respectively, are partially shown. The results indicate the number of bacterial species included in the Genetic name of microorganisms that are clinically important or may be detected in the manufacturing environment, among bacterial species corresponding to database. Regarding bacteria, 815 bacterial species could be detected (Table 2). They included Clostridium sporogenes, Pseudomonas aeruginosa, Staphylococcus aureus, and Bacillus subtilis. Regarding fungi, 506 bacterial species could be detected (Table 3). They included two bacterial species (Aspergillus brasiliensis and Candida albicans) described in JP.

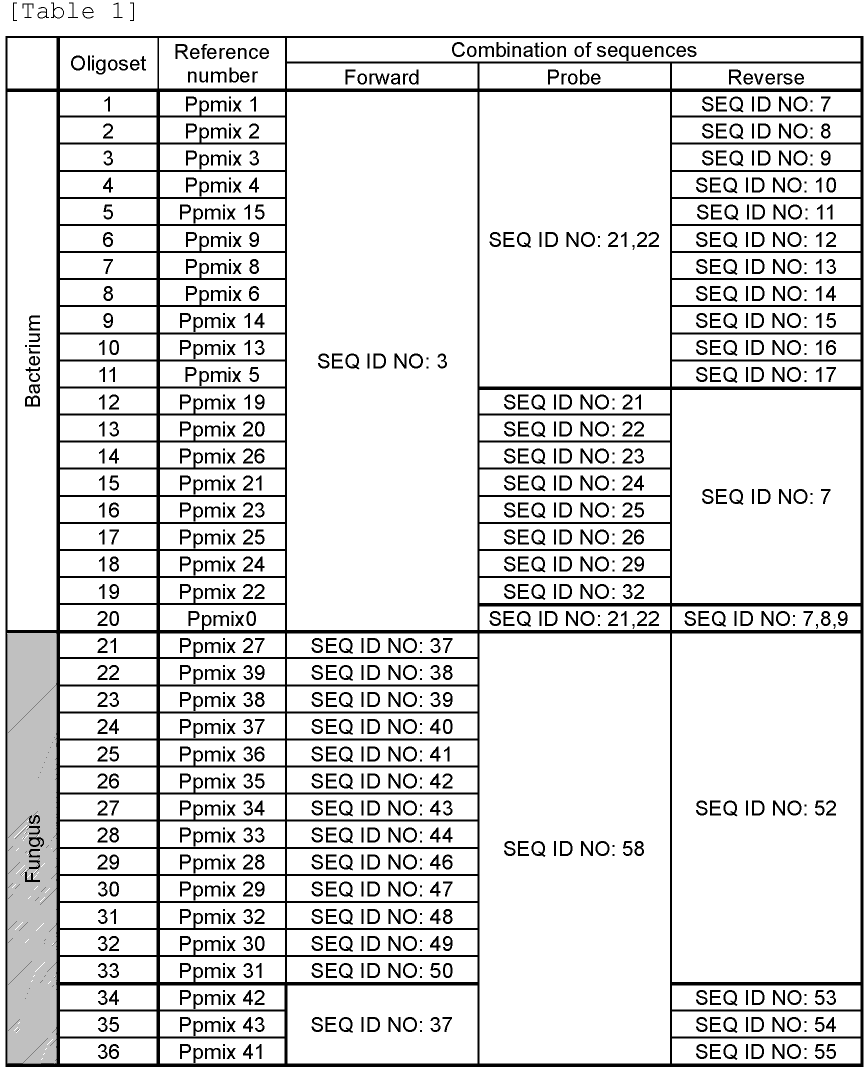

**[Table 2]**

| Generic name | Number of bacterial species | Generic name | Number of bacterial species | Generic name | Number of bacterial species | Generic name | Number of bacterial species |
|---|---|---|---|---|---|---|---|
| *Achromobacter* | 5 | *Curvibacter* | 1 | *Methylorubrum* | 2 | *Rhodococcus* | 3 |
| *Acinetobacter* | 16 | *Delftia* | 1 | *Microbacterium* | 2 | *Roseomonas* | 1 |
| *Actinoalloteichus* | 1 | *Dermacoccus* | 1 | *Micrococcus* | 8 | *Rothia* | 2 |
| *Actinomyces* | 2 | *Dietzia* | 1 | *Moraxella* | 3 | *Rubrobacter* | 1 |
| *Aeromonas* | 2 | *Elizabethkingia* | 1 | *Morganella* | 1 | *Saccharopolyspora* | 1 |
| *Alcaligenes* | 2 | *Embleva* | 1 | *Mycobacterium* | 1 | *Salimicrobium* | 1 |
| *Alicyclobacillus* | 2 | *Empedobacter* | 1 | *Neorhizobium* | 1 | *Salmonella* | 1 |
| *Aliivibrio* | 2 | *Ensifer* | 2 | *Nesterenkonia* | 1 | *Serratia* | 8 |
| *Aneurinibacillus* | 2 | *Enterobacter* | 5 | *Nitrosomonas* | 4 | *Shimwellia* | 1 |
| *Arthrobacter* | 17 | *Enterococcus* | 7 | *Nocardioides* | 1 | *Sphingobacterium* | 4 |
| *Atopobium* | 1 | *Escherichia* | 5 | *Oceanimonas* | 1 | *Sphingomonas* | 4 |
| *Bacillus* | 62 | *Flavobacterium* | 1 | *Oligella* | 1 | *Sporolactobacillus* | 1 |
| *Bradyrhizobium* | 1 | *Geobacillus* | 4 | *Paenarthrobacter* | 5 | *Sporosarcina* | 2 |
| *Brevibacillus* | 9 | *Glutamicibacter* | 4 | *Paenibacillus* | 18 | *Staphylococcus* | 21 |
| *Brevibacterium* | 1 | *Halomonas* | 5 | *Paenialutamicibacter* | 1 | *Stenotrophomonas* | 1 |
| *Brevundimonas* | 2 | *Herbaspirillum* | 1 | *Pantoea* | 1 | *Streptococcus* | 11 |
| *Burkholderia* | 5 | *Hydrogenophaga* | 4 | *Pedobacter* | 1 | *Streptomyces* | 227 |
| *Carnobacterium* | 2 | *lodobacter* | 1 | *Photobacterium* | 1 | *Terrabacter* | 1 |
| *Chromobacterium* | 2 | *Kitasatospora* | 16 | *Phvcicoccus* | 1 | *Terrimonas* | 1 |
| *Chryseobacterium* | 3 | *Klebsiella* | 5 | *Planomicrobium* | 1 | *Thauera* | 1 |
| *Citrobacter* | 7 | *Kluyvera* | 1 | *Pluralibacter* | 1 | *Tsukamurella* | 1 |
| *Clavibacter* | 2 | *Kocuria* | 3 | *Proteus* | 4 | *Ureibacillus* | 1 |
| *Clostridium* | 1 | *Lactobacillus* | 65 | *Providencia* | 1 | *Variovorax* | 1 |
| *Cobetia* | 1 | *Lancefieldella* | 1 | *Pseudarthrobacter* | 2 | *Vibrio* | 36 |
| *Comamonas* | 1 | *Leclercia* | 1 | *Pseudescherichia* | 1 | *Virgibacillus* | 1 |
| *Corvnebacterium* | 29 | *Lelliottia* | 1 | *Pseudomonas* | 49 | *Weissella* | 2 |
| *Cosenzaea* | 1 | *Listonella* | 2 | *Psvchrobacter* | 1 | *Williamsia* | 1 |
| *Cronobacter* | 1 | *Lysinibacillus* | 3 | *Ralstonia* | 2 | *Yersiniae* | 1 |
| *Cupriavidus* | 1 | *Marinobacter* | 1 | *Raoultella* | 3 | *Yinghuangia* | 1 |
| *Curtobacterium* | 1 | *Mesorhizobium* | 5 | *Revranella* | 1 | Total | 815 |
| *Cutibacterium* | 0 | *Methylobacterium* | 3 | *Rhizobium* | 20 | | |

**[Table 3]**

| Generic name | Number of bacterial species | Generic name | Number of bacterial species | Generic name | Number of bacterial species | Generic name | Number of bacterial species |
|---|---|---|---|---|---|---|---|
| *Absidia* | 5 | *Cokeromyces* | 1 | *Moniliella* | 5 | *Rhizopus* | 9 |
| *Acremonium* | 21 | *Cryptococcus* | 6 | *Mortierella* | 7 | *Rhodotorula* | 10 |
| *Ambrosiozyma* | 11 | *Cunninghamella* | 2 | *Mucor* | 15 | *Saccharomyces* | 9 |
| *Apophysomyces* | 1 | *Curvularia* | 1 | *Neotestudina* | 1 | *Scedosporium* | 1 |
| *Arthrinium* | 11 | *Cystobasidium* | 1 | *Nigrospora* | 1 | *Scopulariopsis* | 1 |
| *Arthrographis* | 1 | *Dipodascus* | 5 | *Paracoccidioides* | 1 | *Scytalidium* | 2 |
| *Aspergillus* | 36 | *Epidermophyton* | 1 | *Penicillium* | 23 | *Sporobolomyces* | 6 |
| *Aureobasidium* | 3 | *Exophiala* | 21 | *Pestalotia* | 1 | *Sporothrix* | 2 |
| *Basidiobolus* | 4 | *Filobasidium* | 6 | *Phialophora* | 2 | *Talaromyces* | 20 |
| *Blastomyces* | 1 | *Fonsecaea* | 3 | *Pichia* | 1 | *Thermomyces* | 1 |
| *Candida* | 154 | *Fusarium* | 9 | *Piedraia* | 1 | *Torula* | 1 |
| *Chrysosporium* | 2 | *Geotrichum* | 5 | *Pseudallescheria* | 1 | *Trichoderma* | 10 |
| *Citeromyces* | 2 | *Histoplasma* | 1 | *Pyrenochaeta* | 1 | *Trichophyton* | 9 |
| *Cladophialophora* | 12 | *Leptosphaeria* | 2 | *Rhinocladiella* | 3 | *Trichosporon* | 13 |
| *Cladosporium* | 5 | *Malassezia* | 9 | *Rhinosporidium* | 1 | Total | 506 |
| *Coccidioides* | 2 | *Microsporum* | 2 | *Rhizomucor* | 2 | | |

### [Human nucleic acid]

As human nucleic acid, nucleic acid extracted from Jurkat cells derived from human leukemic T-cells was used. Using an automated nucleic acid extraction device MagNA Pure 24 (Roche Diagnostics K.K.), a solution (final volume: 100 µL)) extracted from Jurkat cell suspension (0.5 mL) of 2.5 × 10⁶ cells/mL was used. The nucleic acid concentration included in the obtained human nucleic acid extract was measured with a micro-volume spectrophotometer NanoDrop (Thermo Fisher Scientific K.K.). The human nucleic acid extract was appropriately diluted to prepare a designated concentration.

As an example, the concentration in a human nucleic acid extract used for a bacterial test was 71.5 ng/µL. Accordingly, it is demonstrated that the concentration is 1.75 × 10⁵ cells per 1000 ng of human nucleic acid, 1.75 × 10⁴ cells per 100 ng of human nucleic acid, and 1.75 × 10³ cells per 10 ng of human nucleic acid.

### [Target nucleic acid]

Regarding the target nucleic acid, a target nucleic acid extract was obtained by nucleic acid extraction from Staphylococcus aureus ATCC29213 for bacterial nucleic acid and from Candida albicans ATCC10231 for fungal nucleic acid.

Subsequently, the number of copies in each of these target nucleic acid extracts was calculated using a calibration curve obtained by measuring standard DNA with known number of copies by a real-time PCR method. Specifically, standard DNA solutions prepared so as to contain 10³, 10⁴, 10⁵, and 10⁶ copies and a target nucleic acid extract diluted by 100-fold were each subjected to measurement by a real-time PCR (Template in Table 4). The real-time PCR conditions are as shown in Tables 4 and 5. A calibration curve was created with the Cq value on the vertical axis and the number of copies (indicated by logarithmic to base 10) on the horizontal axis using four points of the measurement values of the standard DNA, and the number of copies of target nucleic acid was calculated from the Cq value. The target nucleic acid extract was appropriately diluted to prepare a designated number of copies.

As an example, an example of Staphylococcus aureus is shown. The results of real-time PCR are as shown in Table 6, and it is demonstrated that the number of copies in the target nucleic acid extract of Staphylococcus aureus is 1.1 × 10⁶ copies/µL.

**[Table 4]**

| Reagent | Volume |
|---|---|
| Platinum^{™} Taq DNA Polymerase | 0.125 µL |
| 10X PCR Buffer (-MgCl₂) | 2.5 µL |
| 50 mM MgCl₂ | 3 µL |
| dNTP Mix (10 mM each) | 2.5 µL |
| 10xPrimer/Probe Mix | 2.5 µL |
| Template | Specified volume |
| Water | Dilution to a total volume of 25 µL |
| Total | 25 µL |

**[Table 5]**

| Temperature | Time | Note |
|---|---|---|
| 95°C | 60 sec | 1 cycle |
| 95°C | 15 sec | 45 cycles |
| 66°C | 1 sec | |

**[Table 6]**

| No. | Template | Cq value |
|---|---|---|
| 1 | Standard DNA10³ | 28.74 |
| 2 | Standard DNA10⁴ | 25.33 |
| 3 | Standard DNA10⁵ | 21.94 |
| 4 | Standard DNA10⁶ | 19.19 |
| 5 | 100-fold diluted target nucleic acid extract(dual testing) | 25.13 |
| | | 25.44 |

### [Standard DNA]

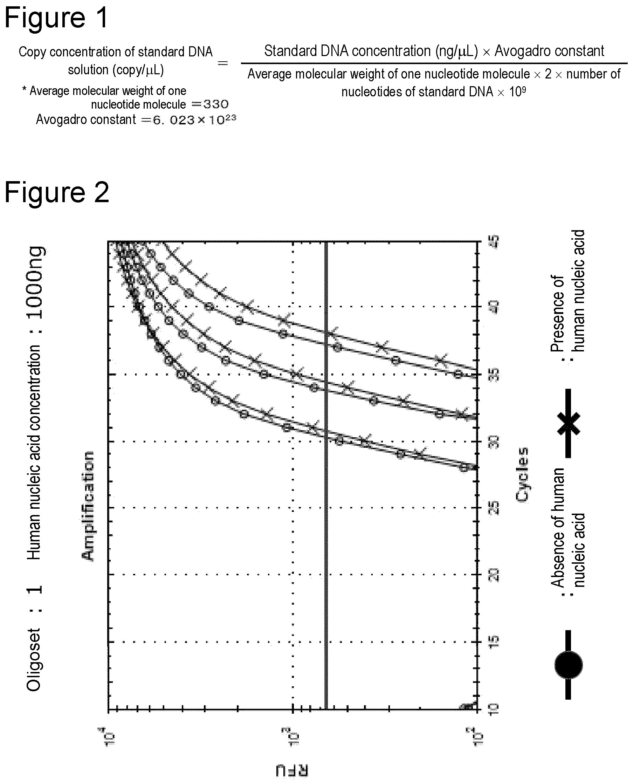

Standard DNA is a synthesized DNA fragment including a nucleic acid amplification region of a primer for bacteria or a primer for fungi used in this test. Outline of the method of producing standard DNA used in this test is as follows. First, a part (SEQ ID NO: 59) of the sequence of Staphylococcus aureus as a bacterium and a part (SEQ ID NO: 60) of the sequence of Candida albicans as a fungus were introduced into respective vectors, followed by gene cloning to obtain a standard DNA solution of a target straight-chain DNA fragment obtained by a restriction enzyme. Subsequently, the concentration of the standard DNA solution was measured with a micro-volume spectrophotometer NanoDrop (Thermo Fisher Scientific K.K.). Furthermore, the number of copies of the standard DNA included in the standard DNA solution (copy concentration of the standard DNA solution) was calculated using the molecular weight of the standard DNA. Specifically, the calculation equation shown in Figure 1 was used.

### [Test method]

The reaction solution to be used in the real-time PCR method was prepared as shown in Table 7. As the combinations of forward primers, probes, and reverse primers (all are manufactured by NIHON TECHNO SERVICE CO., LTD.) used in 10× Primer/Probe Mix, the oligosets shown in Table 1 were used. Regarding the probes, FAM is attached to the 5' end, and BHQ1 is attached to the 3' end. The real-time PCR was performed using a CFX Connect^{™} real-time PCR analysis system (Bio-Rad Laboratories, Inc.) under a reaction condition of heating at 95°C for 10 seconds and then 45 cycles of heating at 95°C for 10 seconds and at 66°C for 60 seconds (Table 8).

**[Table 7]**

| Reagent | Volume |
|---|---|
| Platinum^{™} Taq DNA Polymerase, DNA-free | 0.25 µL |
| 10X PCR Buffer (-MgCl₂) | 2.5 µL |
| 50 mM MgCl₂ | 0.75 µL |
| dNTP Mix (10 mM each) | 0.5 µL |
| 10xPrimer/Probe Mix | 2.5 µL |
| Target nucleic acid | 0 copy |
| | 10 copies |
| | 100 copies |
| | 1000 copies |
| Human nucleic acid | 0ng |
| | 1000ng |
| Water | Dilution to a total volume of 25 µL |
| Total | 25 µL |

**[Table 8]**

| Temperature | Time | Note |
|---|---|---|
| 95°C | 10 sec | 1 cycle |
| 95°C | 10 sec | 45 cycles |
| 66°C | 60 sec | |

### [Method for judging inhibition of nucleic acid amplification reaction]

When real-time PCR of 3 or more samples with the target nucleic acid amounts at equal increments is performed, the amplification curves thereof are thought to be approximately equally spaced. For example, when real-time PCR is performed using samples prepared such that the concentrations of target nucleic acid are adjusted in 10-fold increments such as 10 copies, 100 copies, and 1000 copies, the amplification curves thereof are almost equally spaced from the right. This means that a calibration curve can be created using the Cq values and number of copies of the real-time PCR.

Furthermore, inhibition of the nucleic acid amplification reaction of target nucleic acid can be recognized by a shift of the amplification curve of the nucleic acid amplification reaction to the right. Because when the nucleic acid amplification reaction of target nucleic acid is inhibited, the Cq value of the nucleic acid amplification reaction becomes larger. Accordingly, when the amplification curve of a sample added with human nucleic acid shifted to the right side compared to the amplification curve of a sample not added with human nucleic acid, it is possible to judge that the nucleic acid amplification reaction of target nucleic acid was inhibited by human nucleic acid. For example, when the amplification curve in real-time PCR of a sample containing 1000 copies of target nucleic acid including human nucleic acid is present near the amplification curve in real-time PCR of a sample containing 100 copies of target nucleic acid not including human nucleic acid, it was judged that the detection sensitivity had dropped by about one-tenth. In addition, for example, when the amplification curve in real-time PCR of a sample containing 1000 copies of target nucleic acid including human nucleic acid is present near the amplification curve in real-time PCR of a sample containing 10 copies of target nucleic acid not including human nucleic acid, it was judged that the detection sensitivity had dropped to about one-hundredth by addition of human nucleic acid. The inhibition effect on the nucleic acid amplification reaction was judged based on the criteria shown in Table 9.

**[Table 9]**

| | |
|---|---|
| +++ | A reduction in detection sensitivity by about 1000 times or more (amplification curve of 1000 copies does not rise) |
| ++ | A reduction in detection sensitivity by about 100 times |
| + | A reduction in detection sensitivity by about 10 times |
| - | No influence on detection sensitivity |

### [Results]

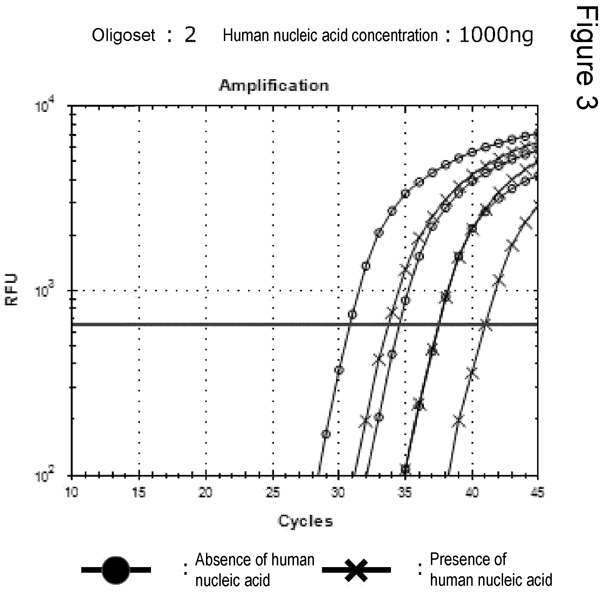

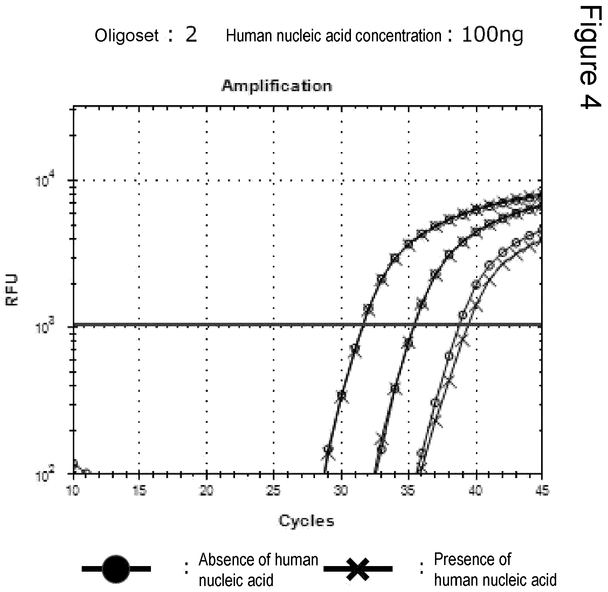

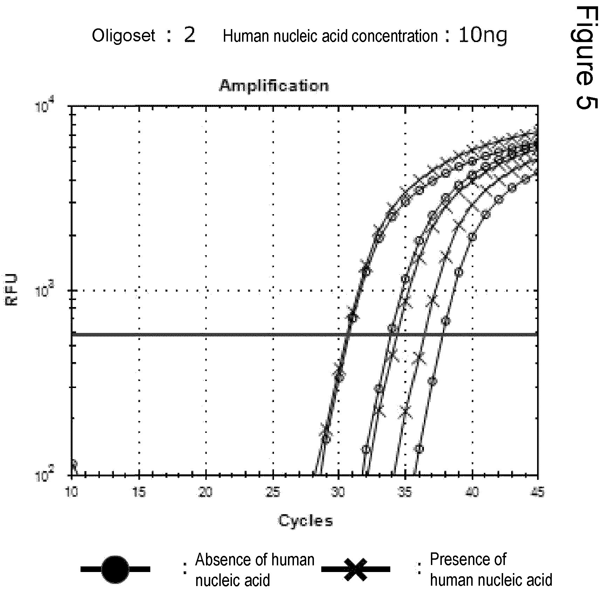

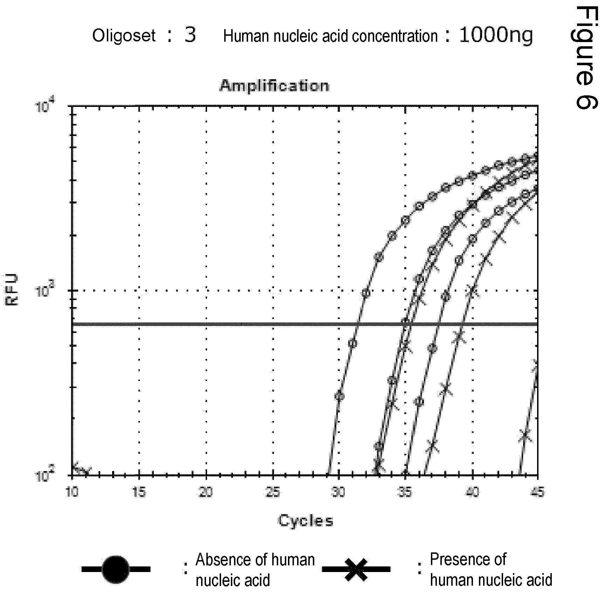

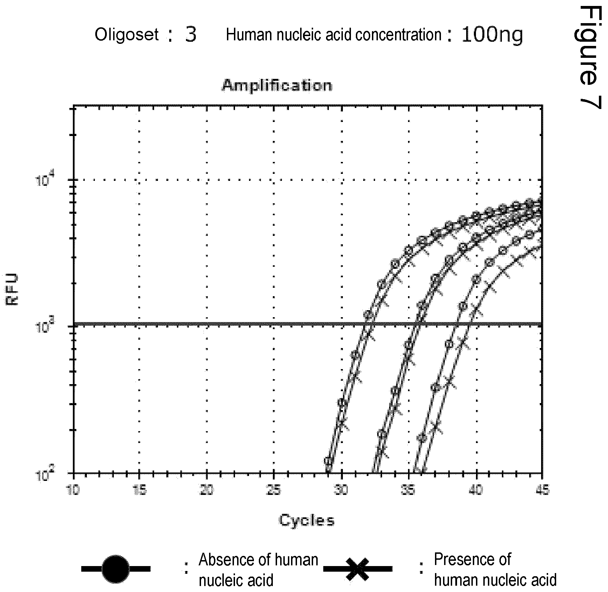

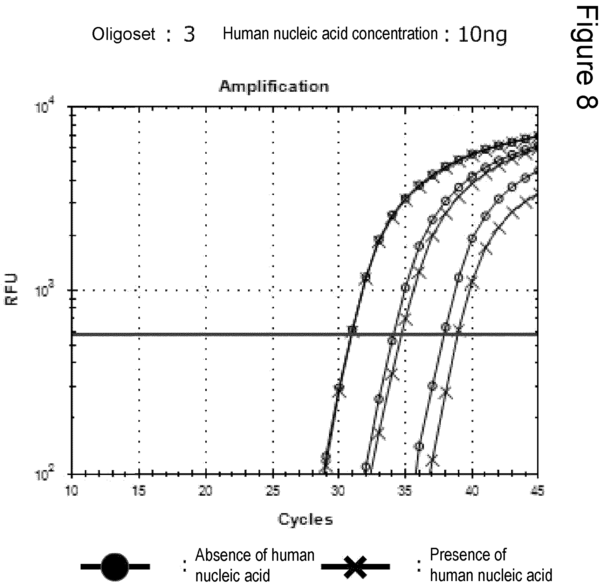

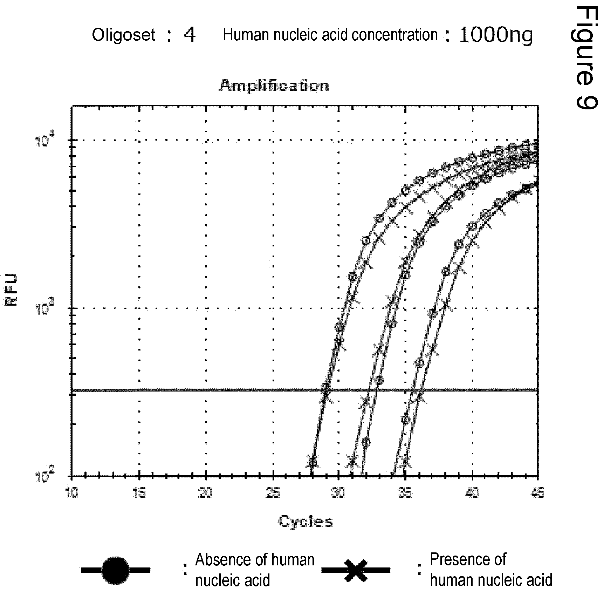

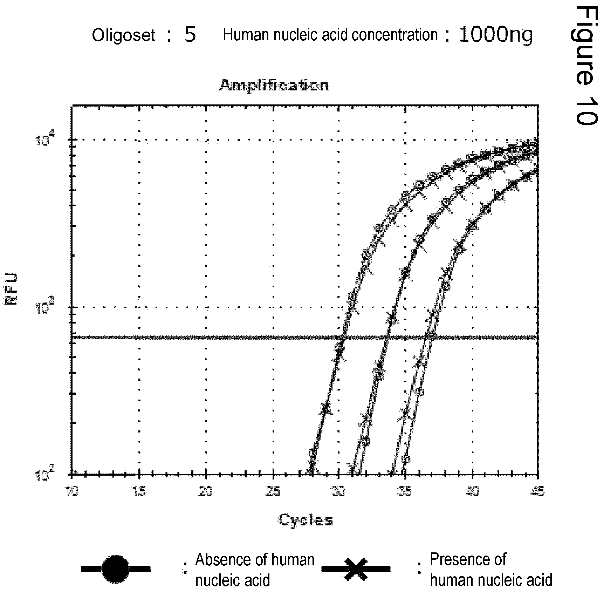

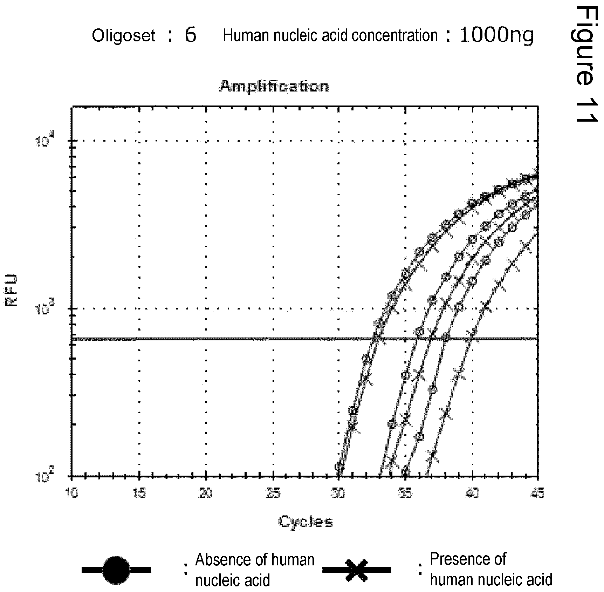

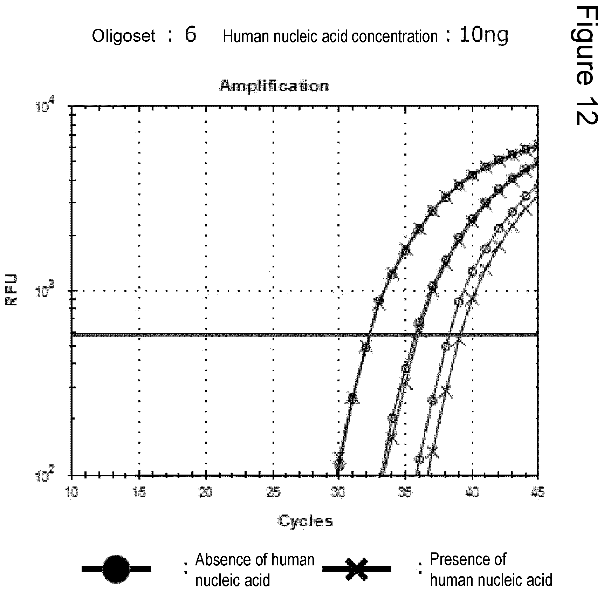

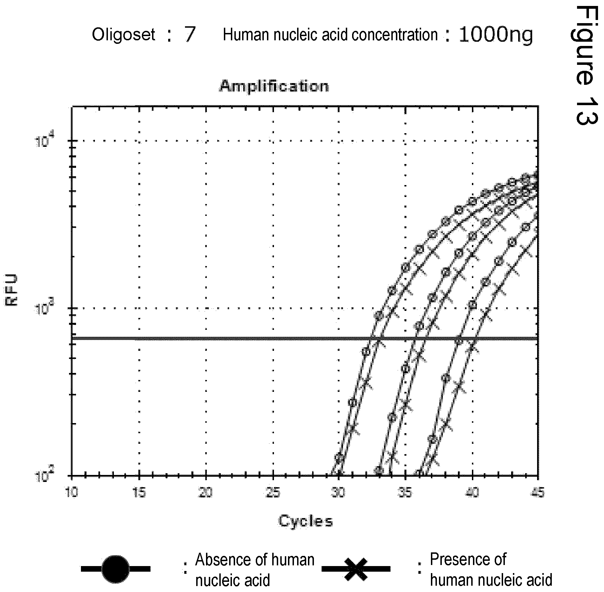

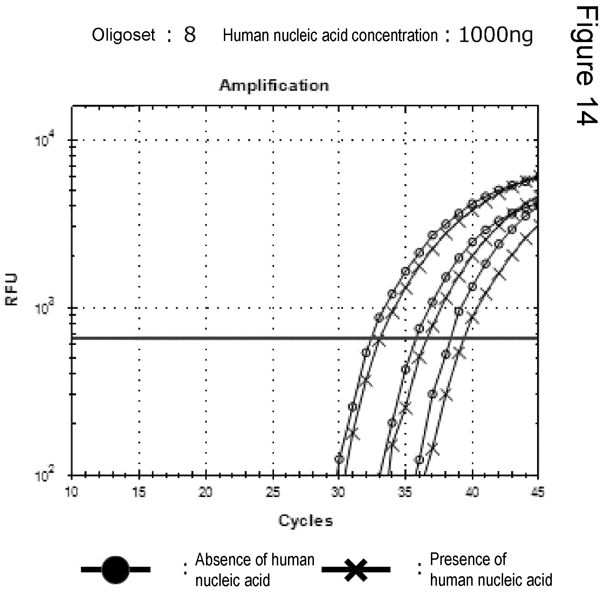

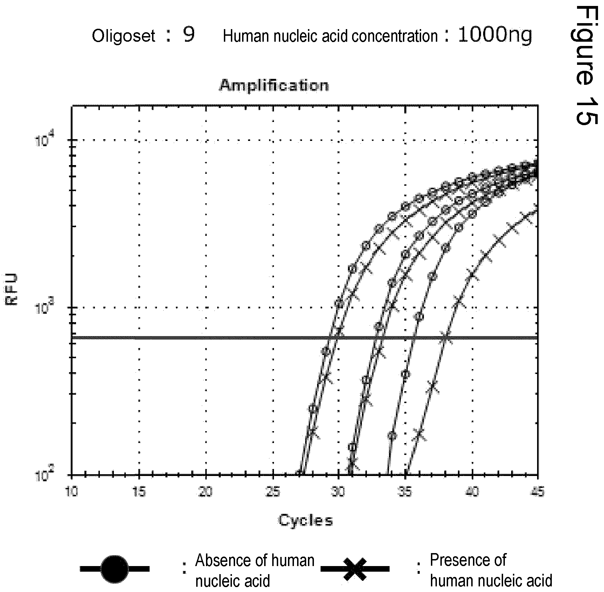

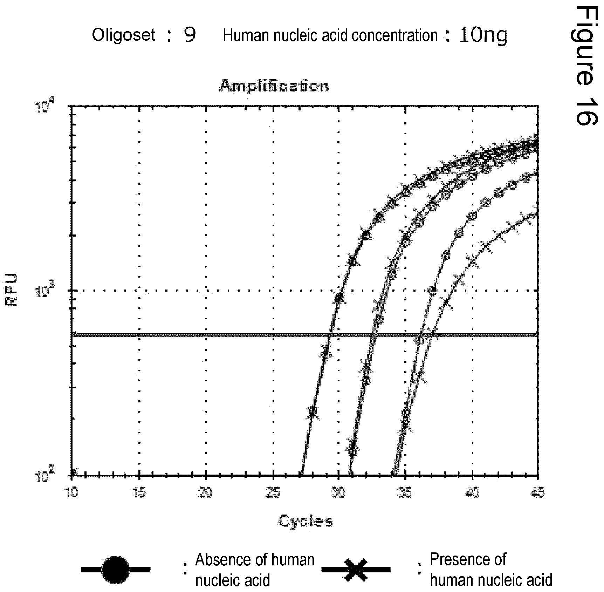

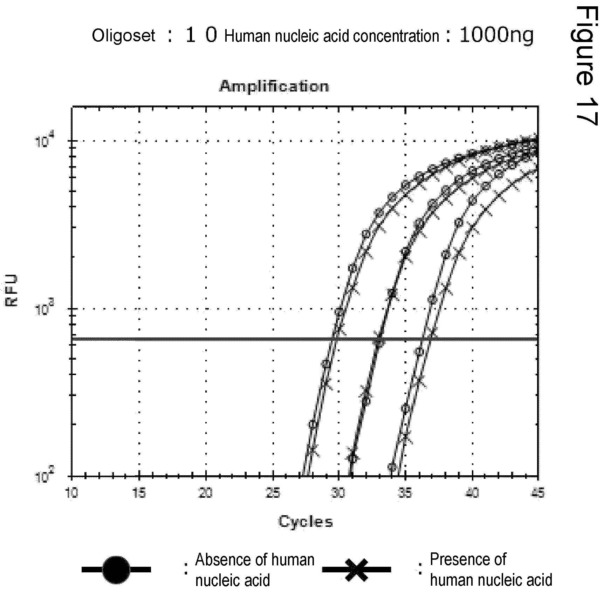

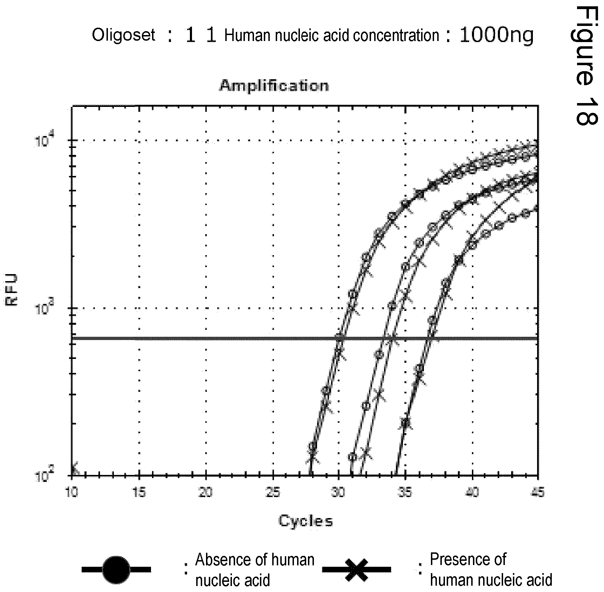

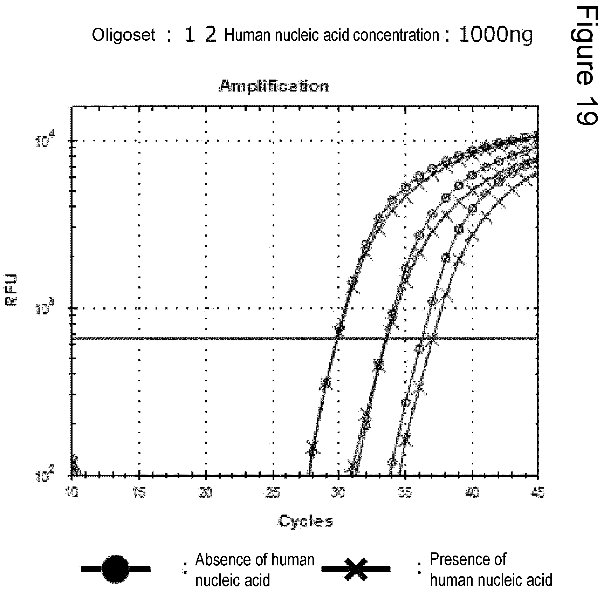

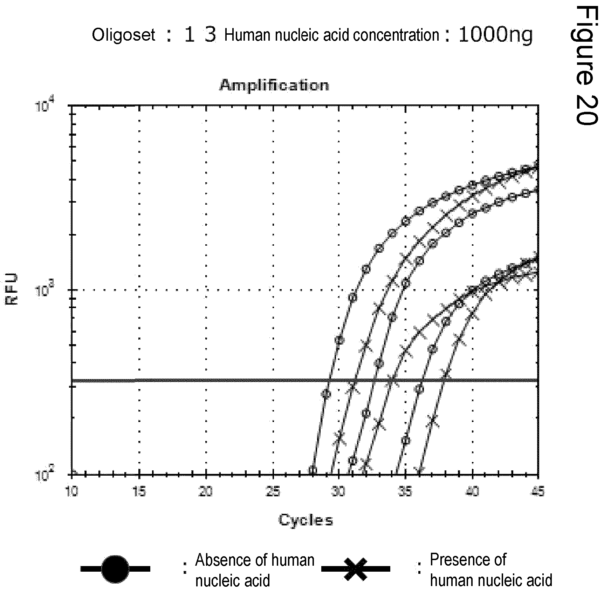

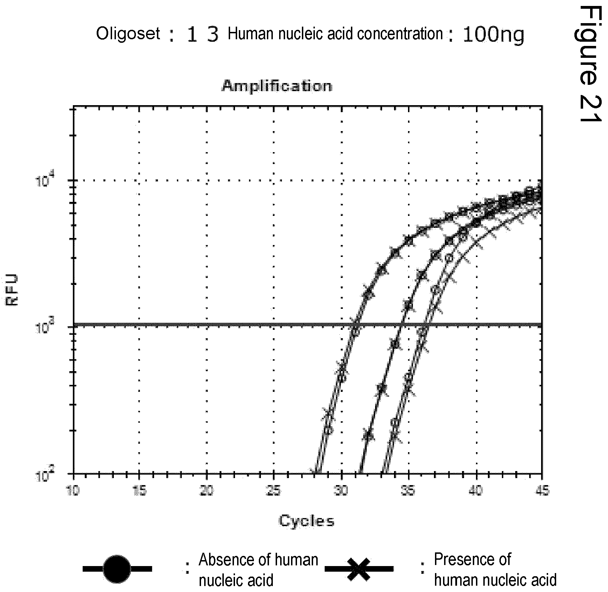

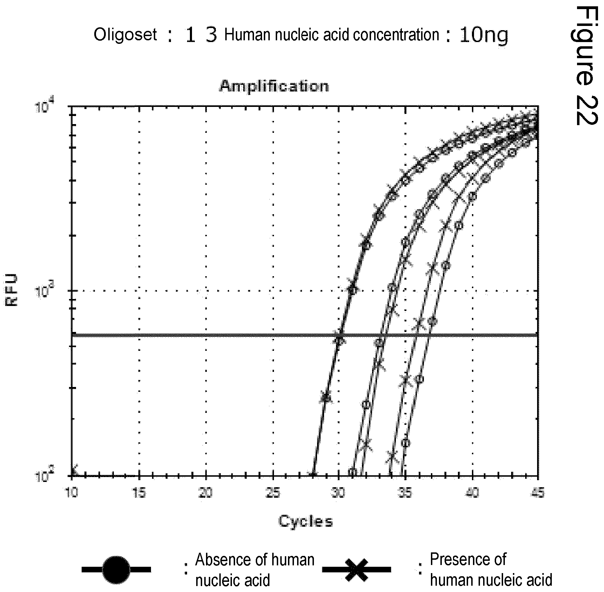

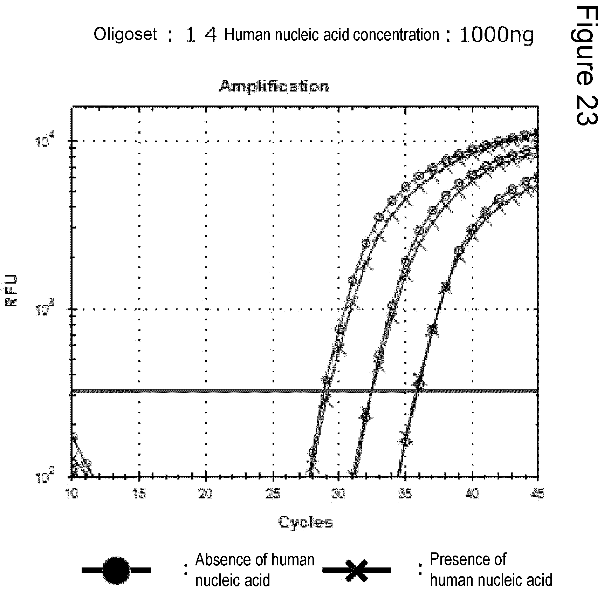

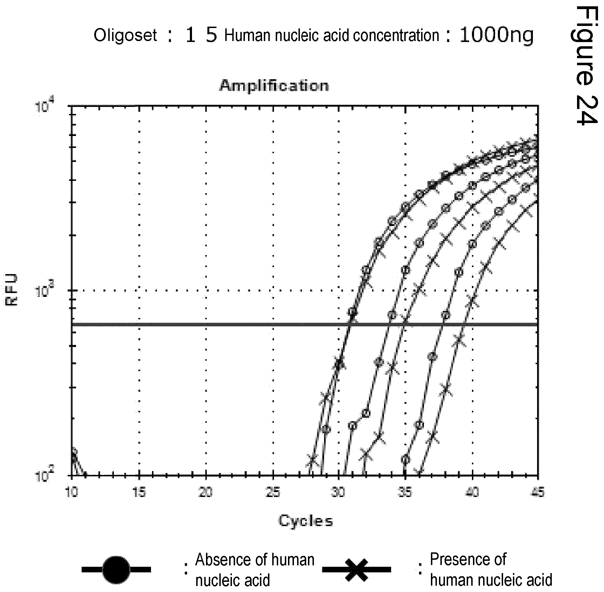

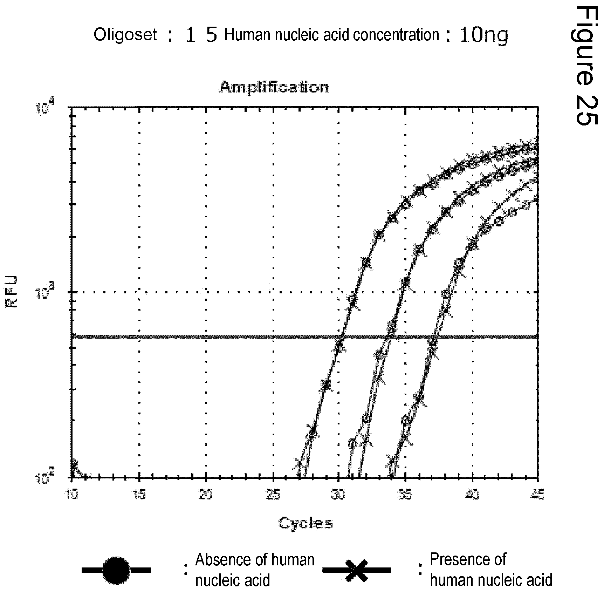

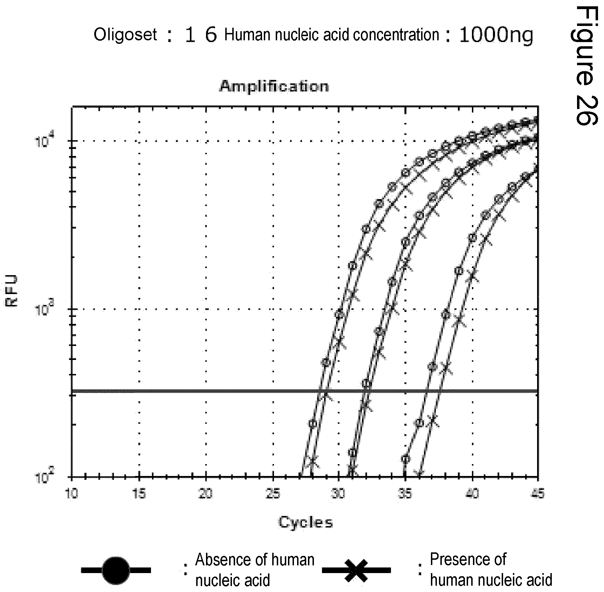

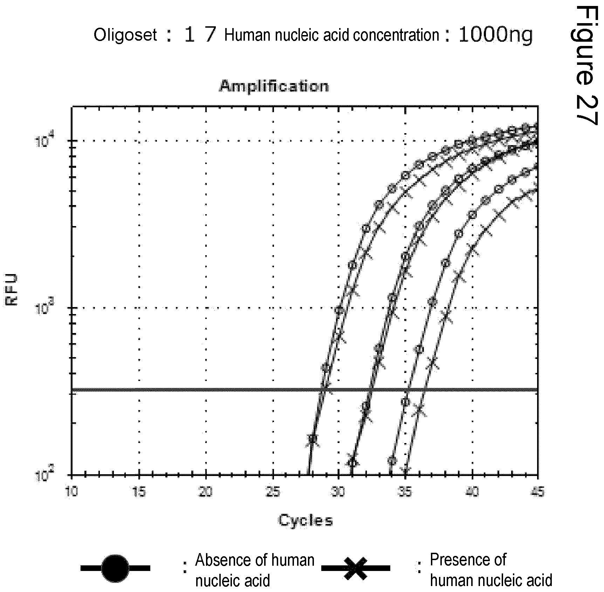

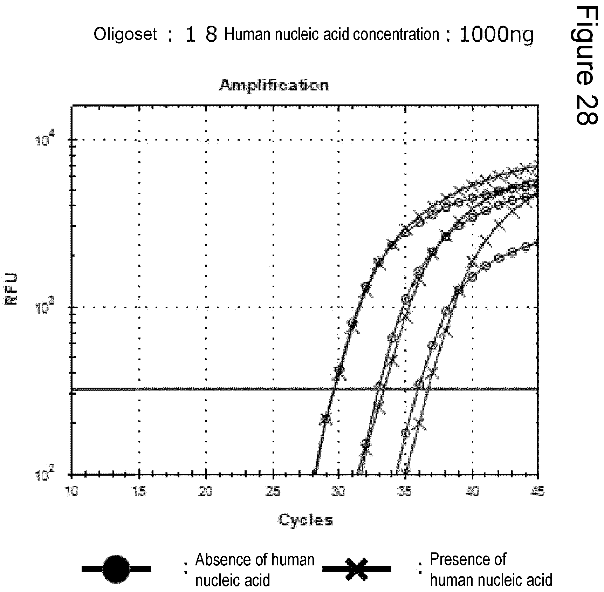

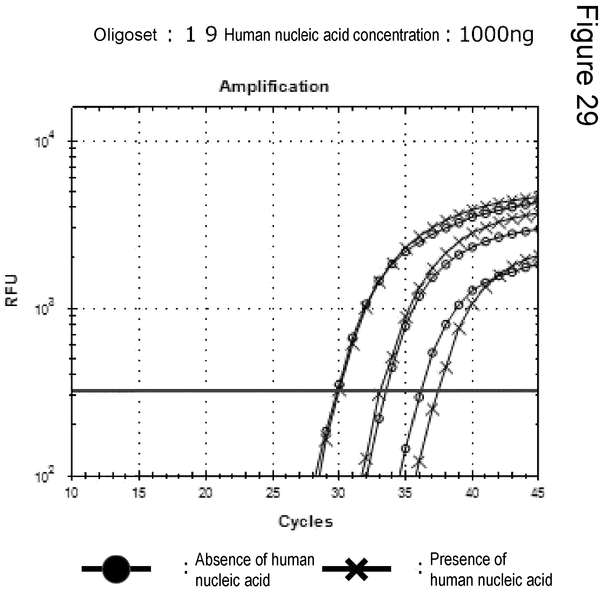

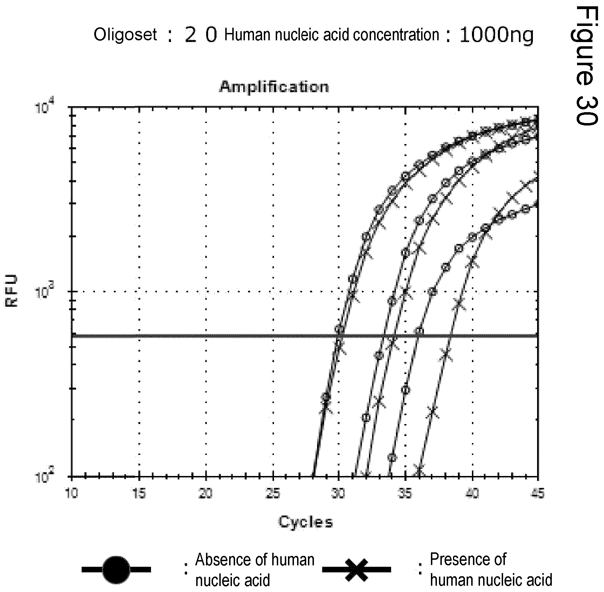

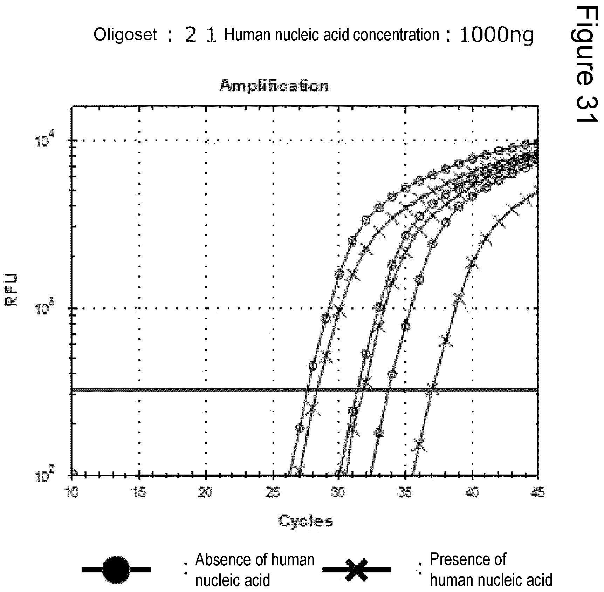

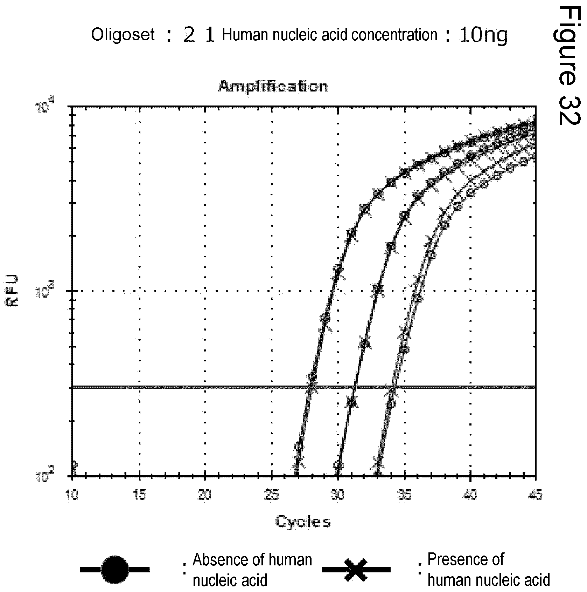

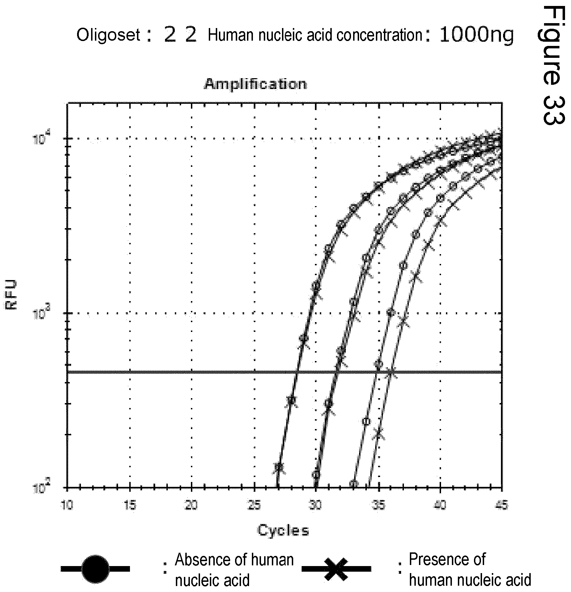

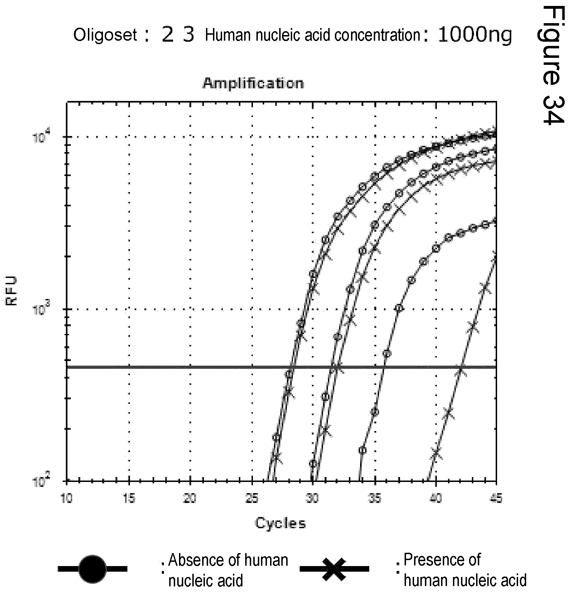

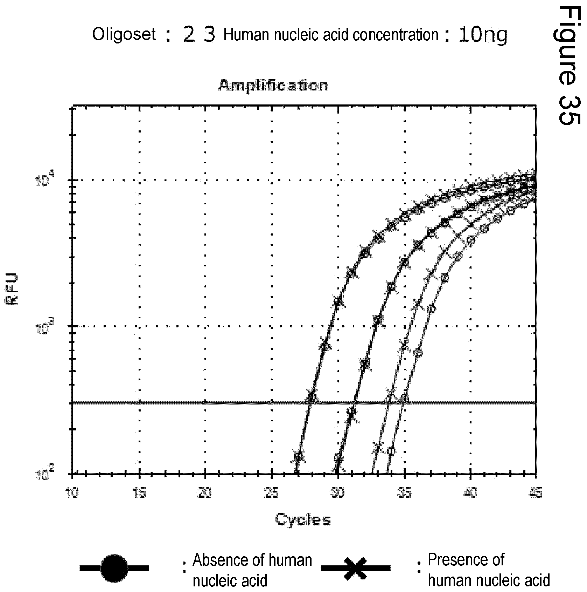

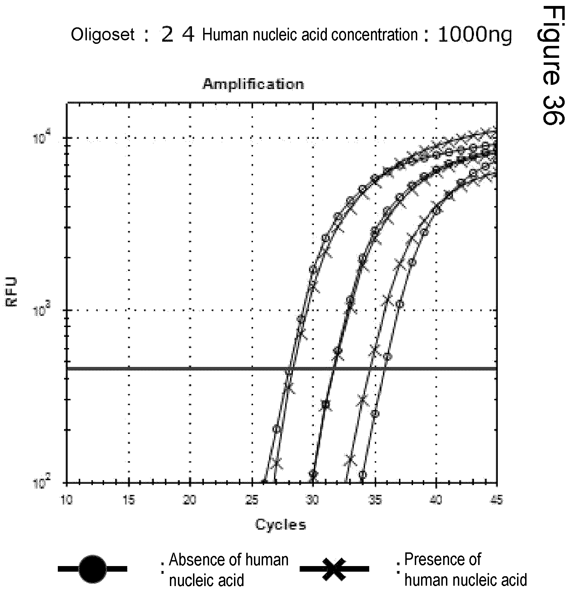

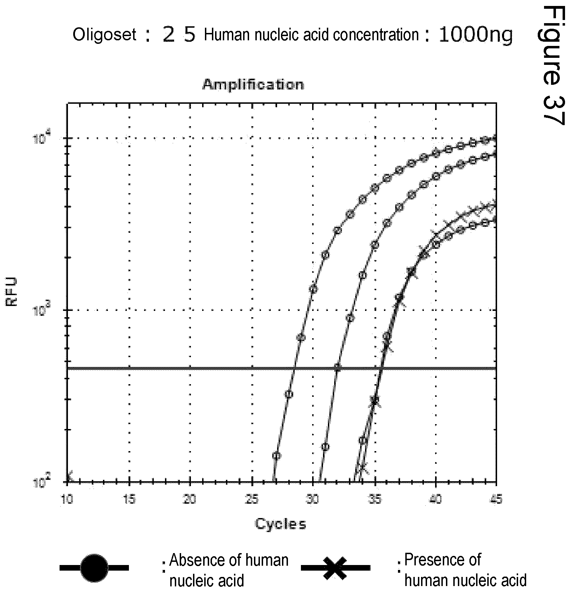

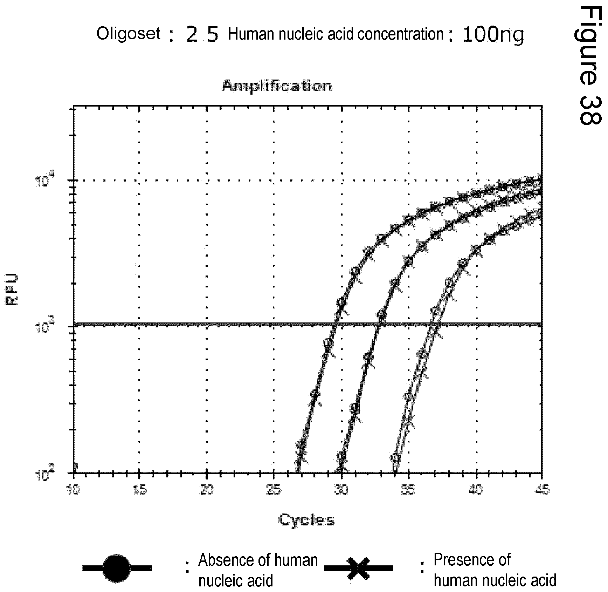

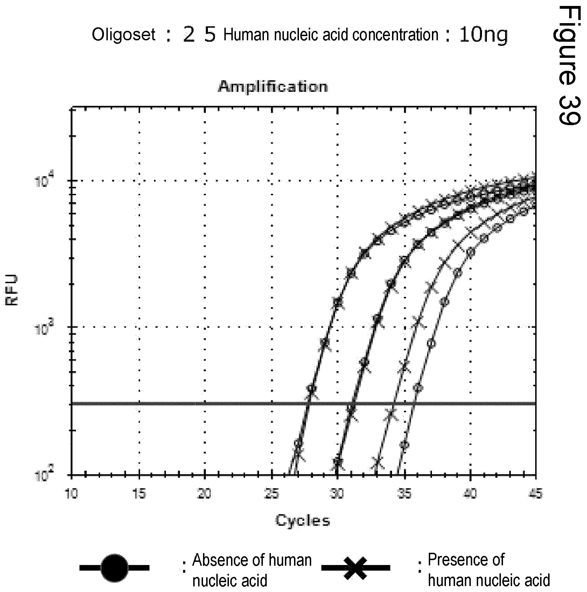

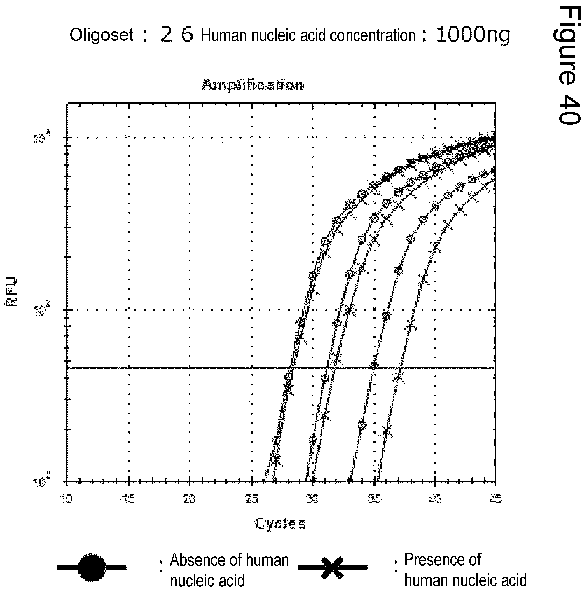

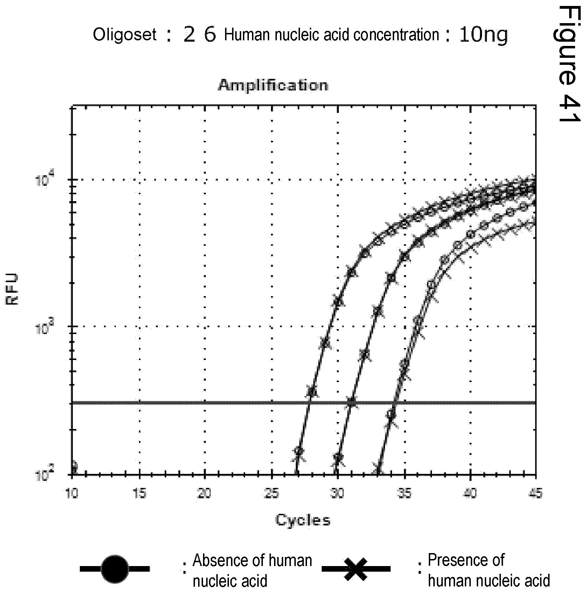

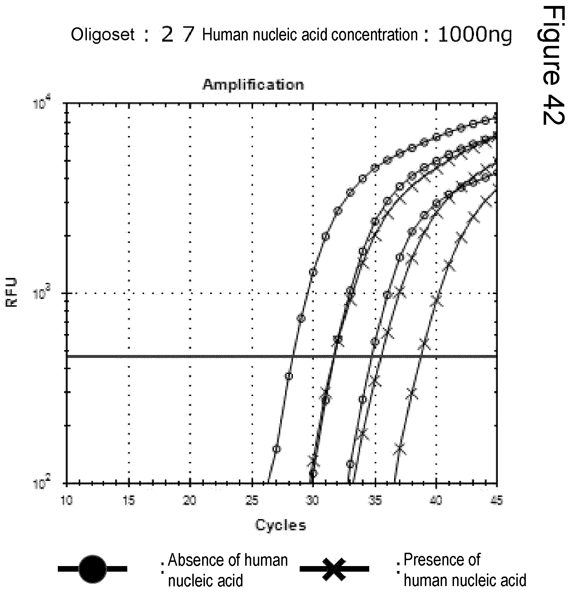

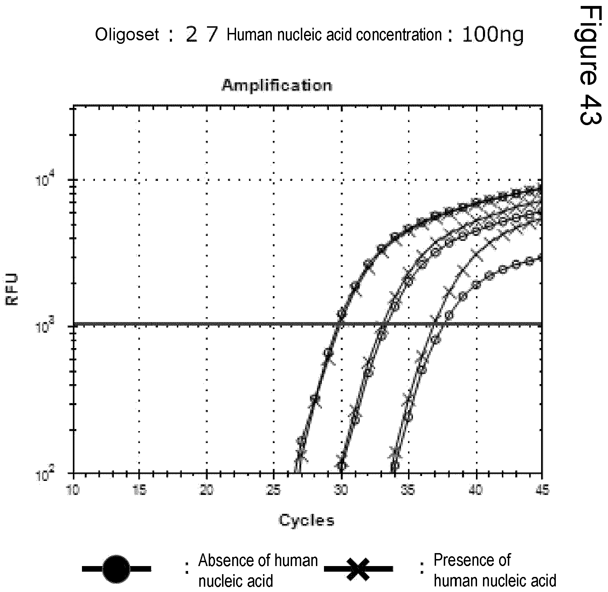

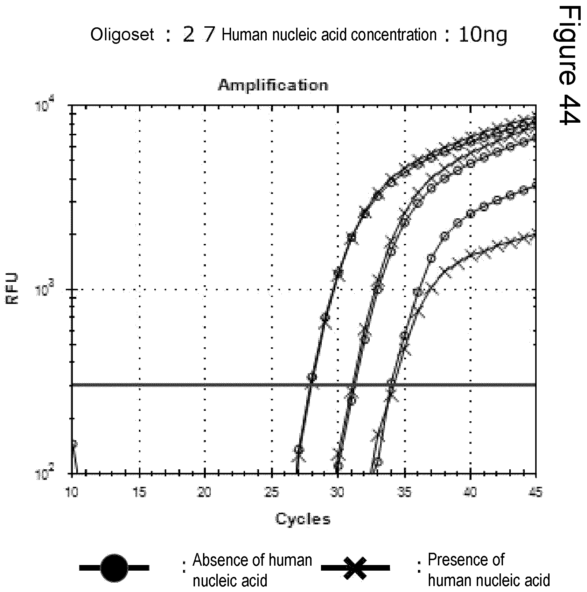

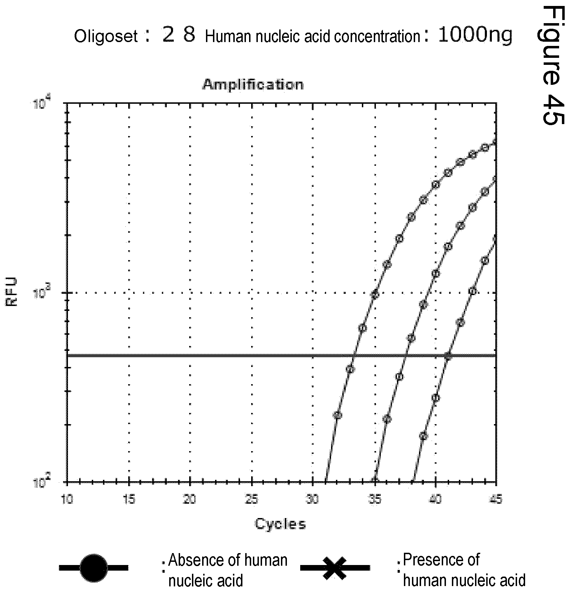

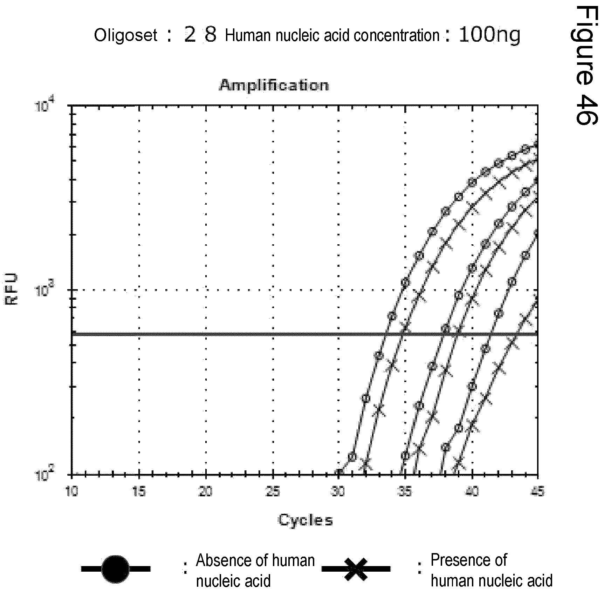

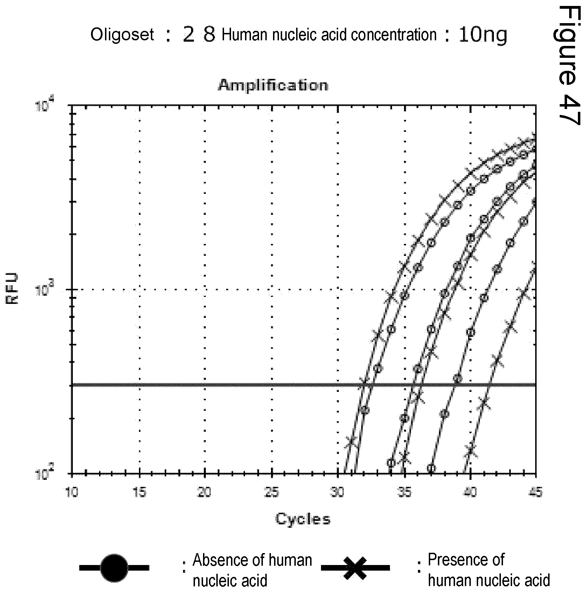

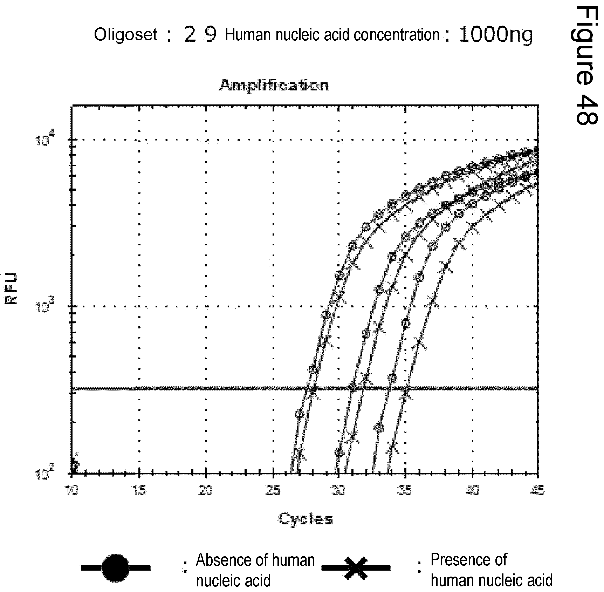

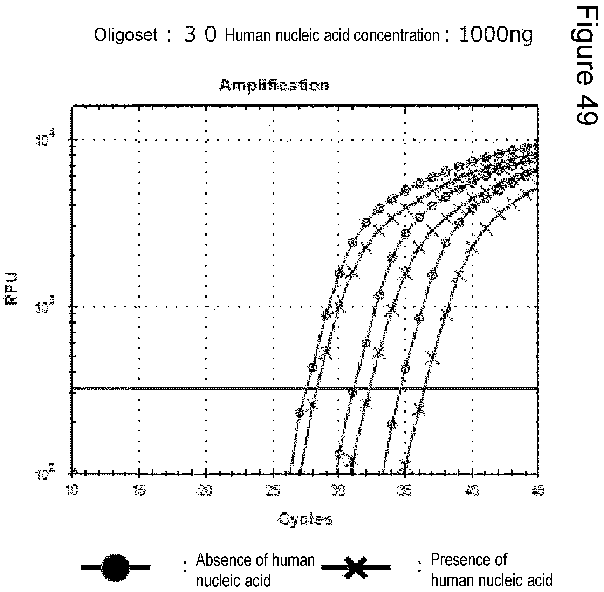

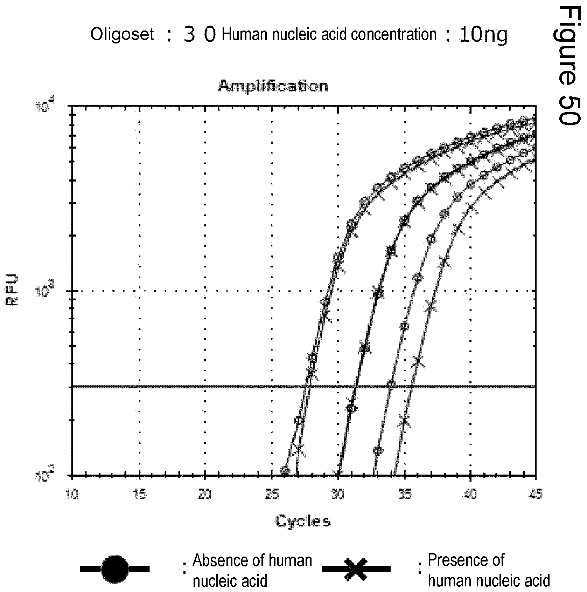

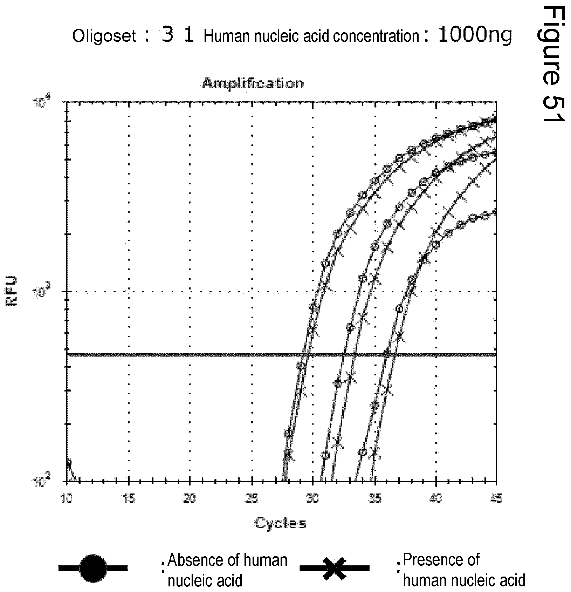

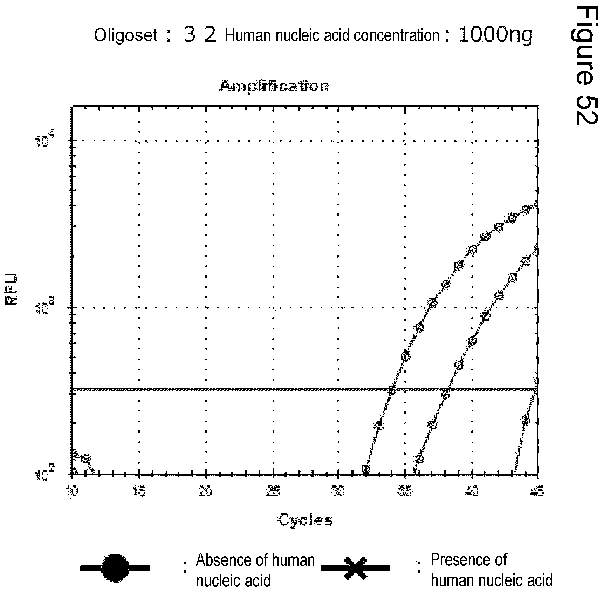

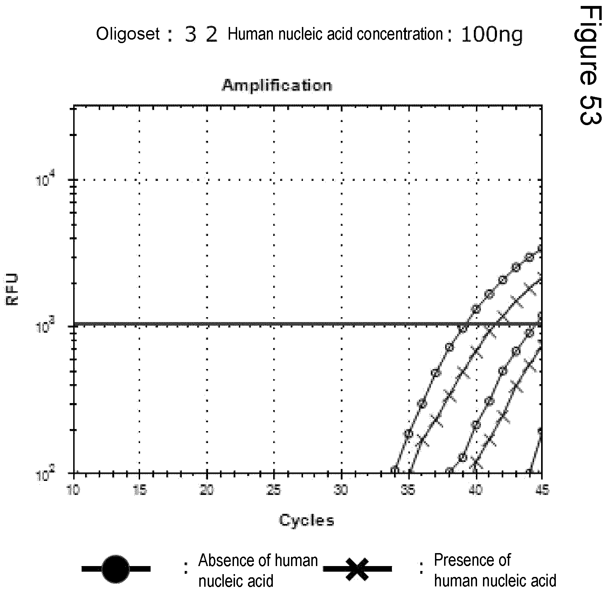

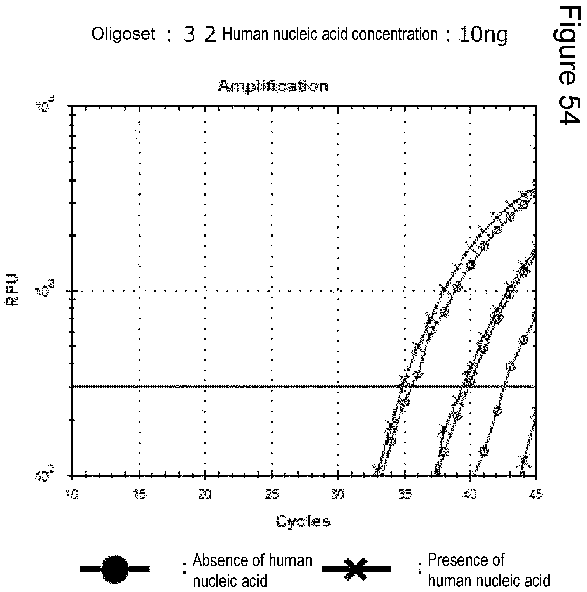

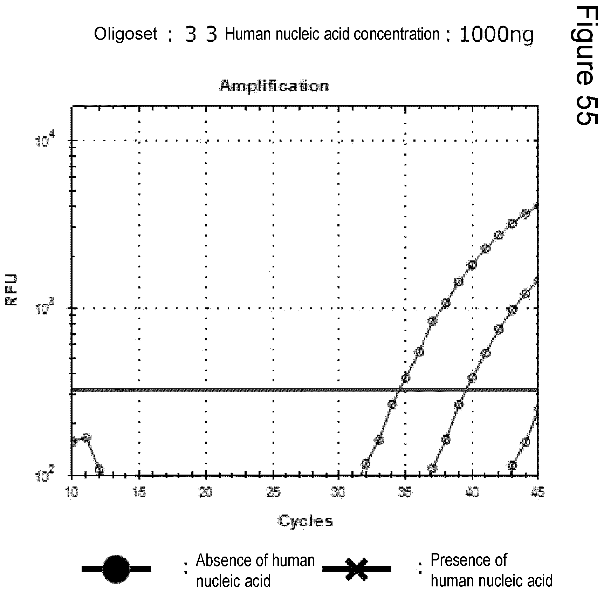

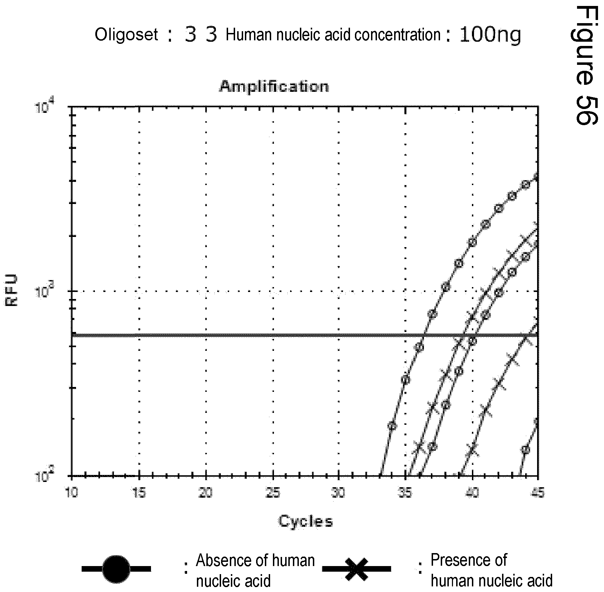

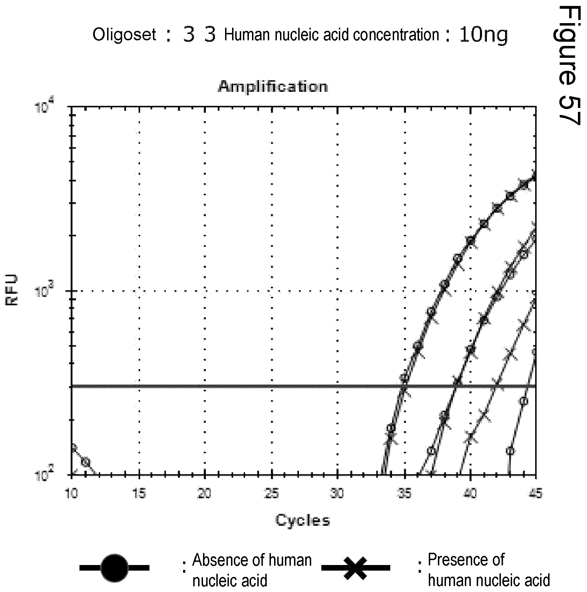

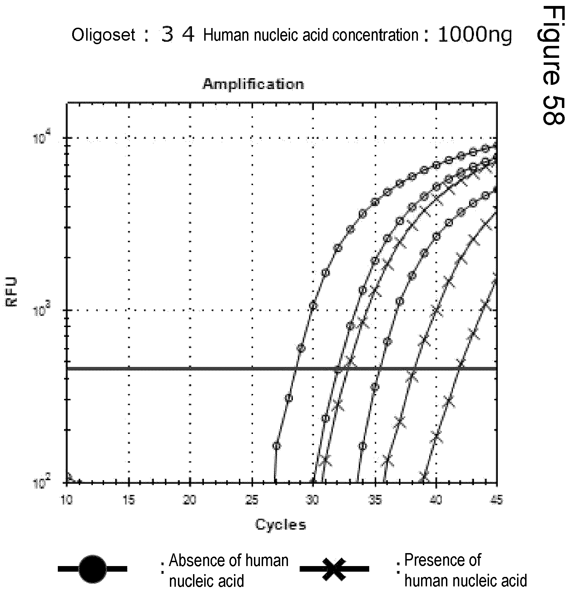

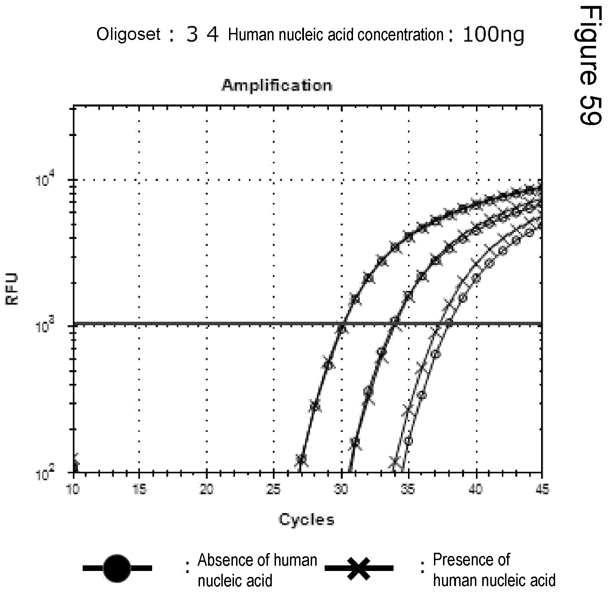

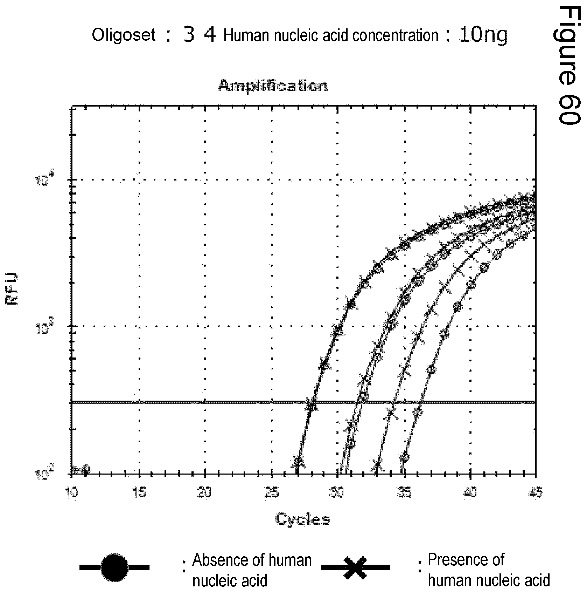

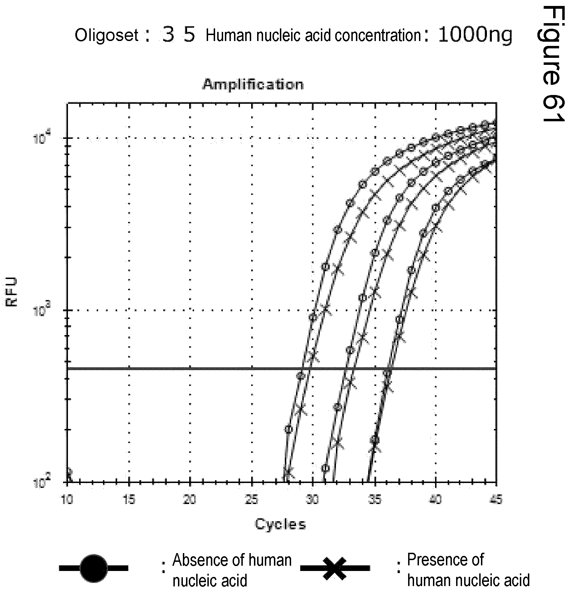

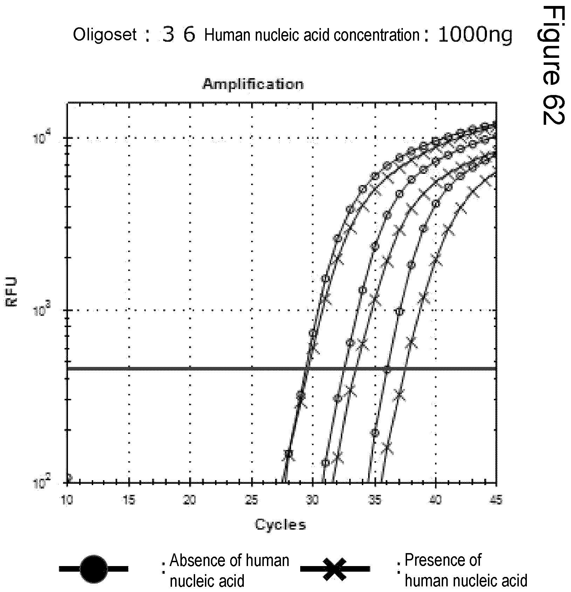

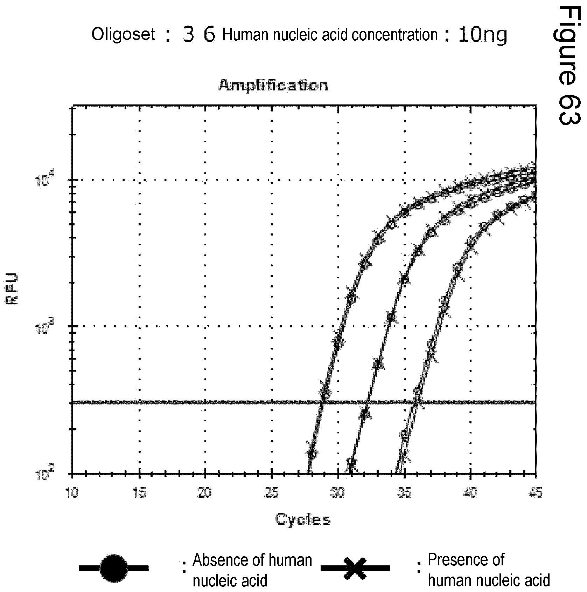

Comparison of primers and probes used in each oligoset is shown in Table 10. Amplification curves using each oligoset are shown in Figures 2 to 63, the inhibition effect on the nucleic acid amplification reaction is shown in Table 11, and possibility of rapid sterility test in the presence of human nucleic acid is shown in Table 12. The nucleotide sequences of probes and reverse primers in oligosets 1 to 20 were excerpted in Table 13, and the nucleotide sequences of forward primers and reverses primers in oligosets 21 to 36 were excerpted in Table 14.

In Figures 2 to 63, amplification curves in real-time PCR of samples not added with human nucleic acid are indicated by "•", and amplification curves in real-time PCR of samples added with human nucleic acid are indicated by "×". The amplification curves were those of 1000 copies, 100 copies, and 10 copies from the left. Furthermore, in Table 11, "Possible" indicates that detection of target nucleic acid was possible, "Impossible" indicates that the detection was impossible, and "Influence" shows the inhibition effect on nucleic acid amplification reaction. Furthermore, in Table 12, "Detected" indicates that the presence of target nucleic acid was detected by the rapid sterility test in the presence of human nucleic acid, and "Undetected" indicates that the presence of target nucleic acid was not detected by the rapid sterility test.

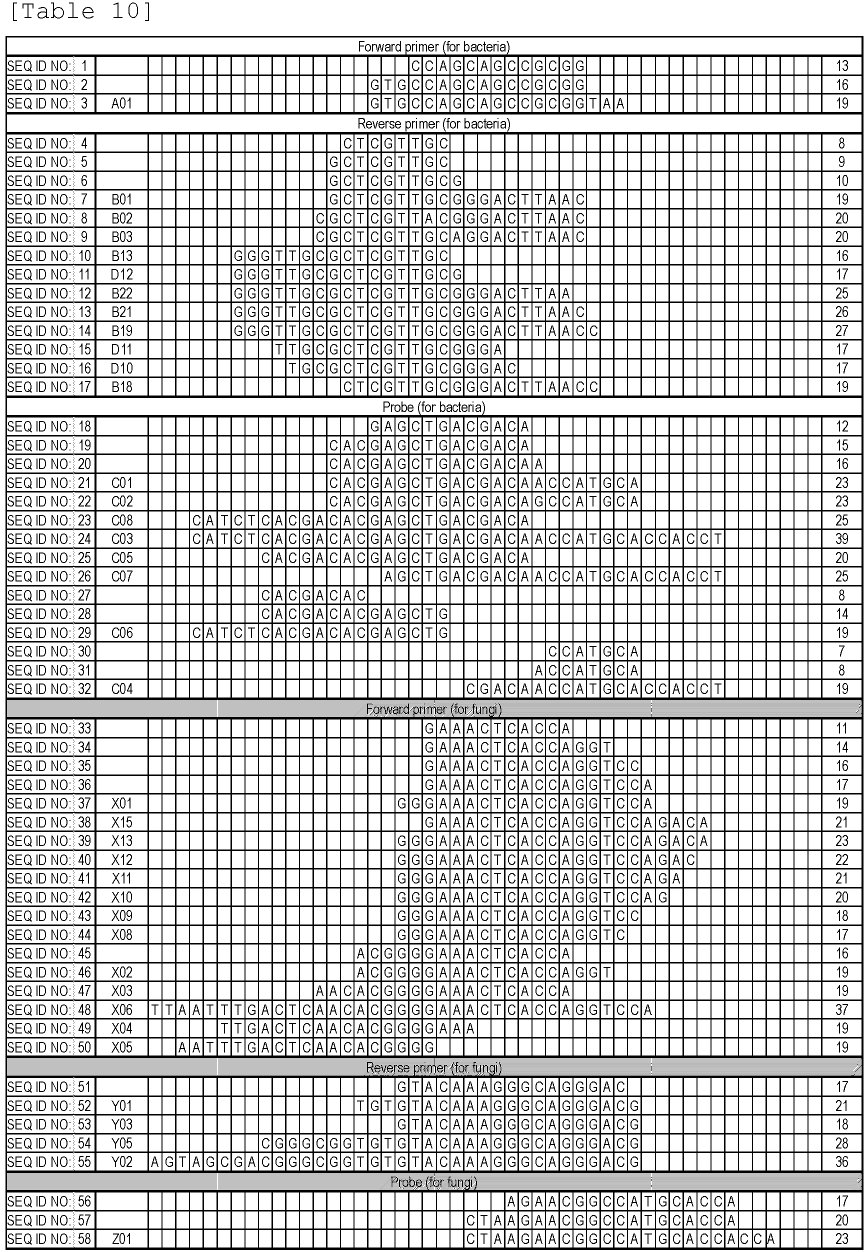

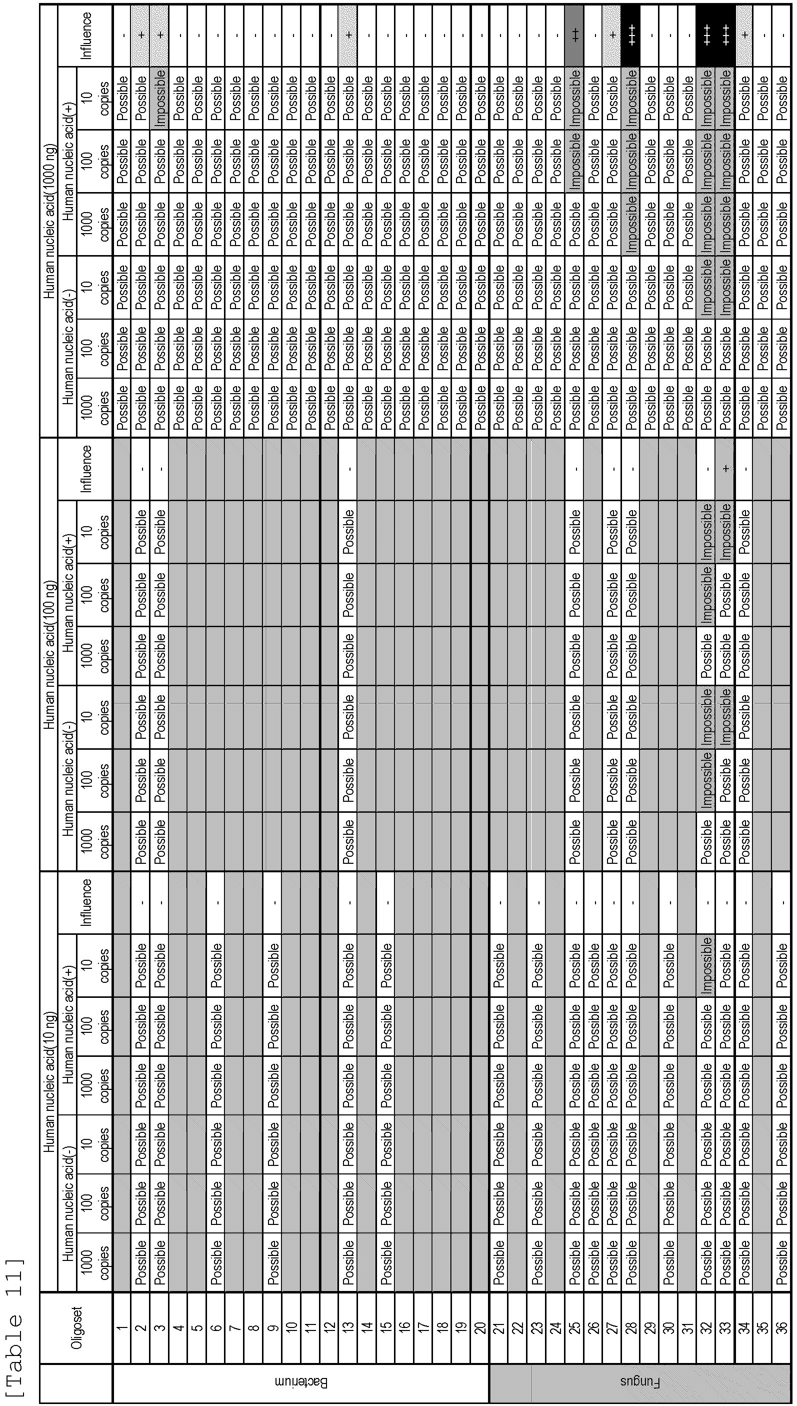

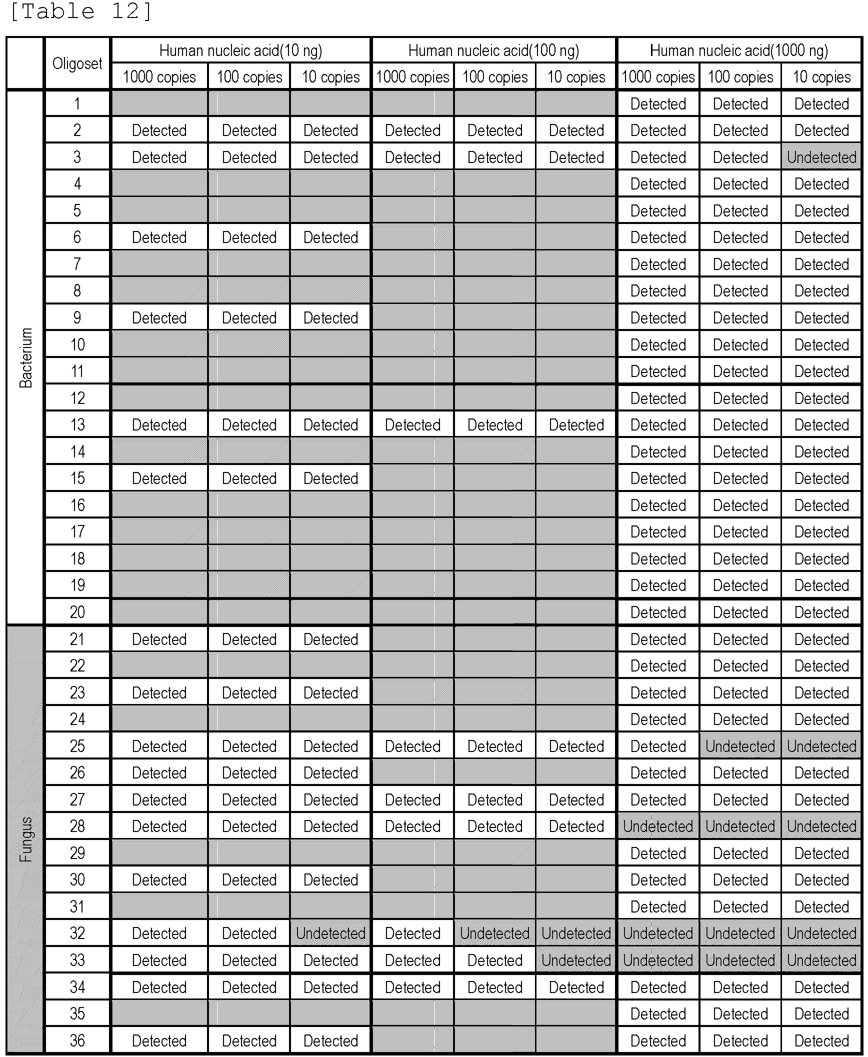

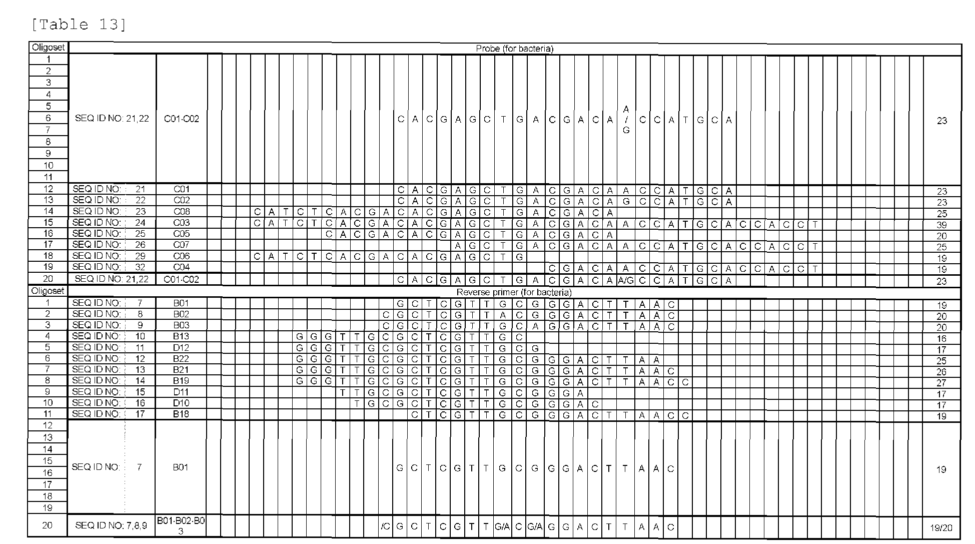

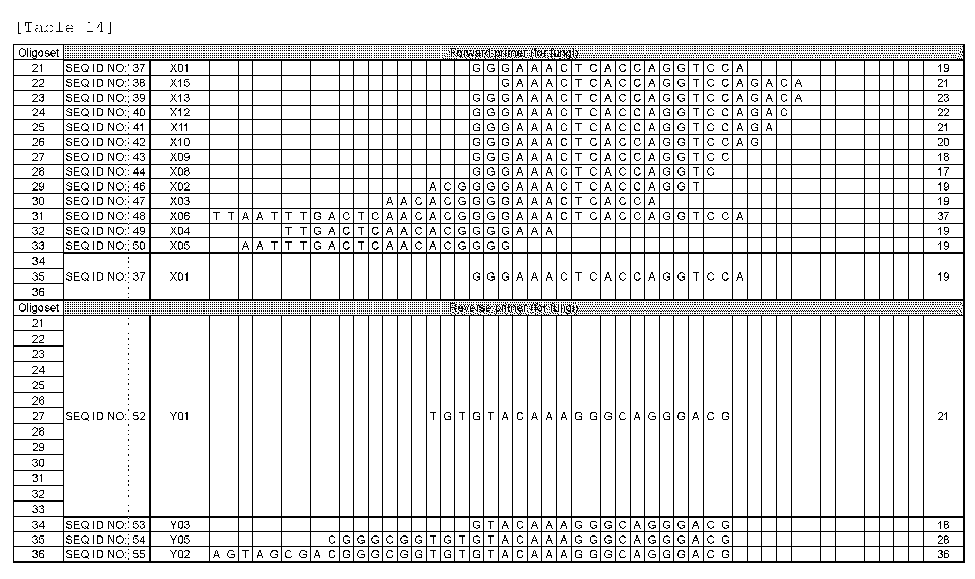

### (Results of bacteria)

In oligosets 1 to 20, 10 copies of target nucleic acid could be detected when no human nucleic acid was added. The results when human nucleic acid was added are shown below.

### i) Possibility of sterility test (Table 12)

In oligosets 1 to 20, 100 copies of target nucleic acid could be detected when 1000 ng of human nucleic acid was added. Furthermore, in oligosets 1 to 2 and oligosets 4 to 20, 10 copies of target nucleic acid could be detected when 1000 ng of human nucleic acid was added. In oligoset 3, although 10 copies of target nucleic acid could not be detected when 1000 ng of human nucleic acid was added, 10 copies of target nucleic acid could be detected when 100 ng of human nucleic acid was added.

From the above, it was demonstrated that even if human nucleic acid is present, oligosets 1 to 20 can be used in rapid sterility tests.

### ii) Effect on nucleic acid amplification reaction inhibition (Table 11)

In oligoset 1, oligosets 4 to 12, and oligosets 14 to 20, even if human nucleic acid was added, the detection sensitivity of the nucleic acid amplification reaction was not influenced. In oligoset 2, oligoset 3, and oligoset 13, the detection sensitivity of the nucleic acid amplification reaction decreased by about 10 times by addition of human nucleic acid.

### (Results of fungi)

In oligosets 21 to 31 and oligosets 34 to 36, 10 copies of target nucleic acid could be detected when no human nucleic acid was added. In oligoset 32, when no human nucleic acid was added, although 10 copies of target nucleic acid could not be detected, 1000 copies of target nucleic acid could be detected. In oligoset 33, when no human nucleic acid was added, although 10 copies of target nucleic acid could not be detected, 100 copies of target nucleic acid could be detected. The results when human nucleic acid was added are shown below.

### i) Possibility of sterility test (Table 12)

In oligosets 21 to 24, oligoset 26, oligoset 27, oligosets 29 to 31, and oligosets 34 to 36, 10 copies of target nucleic acid could be detected when 1000 ng of human nucleic acid was added. In oligoset 25, when 1000 ng of human nucleic acid was added, although 100 copies of target nucleic acid could not be detected, 1000 copies of target nucleic acid could be detected. In oligoset 25, 10 copies of target nucleic acid could be detected when 100 ng of human nucleic acid was added. In oligoset 28, although 1000 copies of target nucleic acid could not be detected when 1000 ng of human nucleic acid was added, 10 copies of target nucleic acid could be detected when 100 ng of human nucleic acid was added. In oligoset 33, 1000 copies of target nucleic acid could not be detected when 1000 ng of human nucleic acid was added. In oligoset 33, when 100 ng of human nucleic acid was added, although 10 copies of target nucleic acid could not be detected, 100 copies of target nucleic acid could be detected. In oligoset 32, 1000 copies of target nucleic acid could not be detected when 1000 ng of human nucleic acid was added. In oligoset 32, when 100 ng of human nucleic acid was added, although 100 copies of target nucleic acid could not be detected, 1000 copies of target nucleic acid could be detected. Furthermore, in oligoset 32, when 10 ng of human nucleic acid was added, although 10 copies of target nucleic acid could not be detected, 100 copies of target nucleic acid could be detected.

From the above, it was demonstrated that even if human nucleic acid is present, oligosets 21 to 36 can be used in rapid sterility tests.

### ii) Effect on nucleic acid amplification reaction inhibition (Table 11)

In oligosets 21 to 24, oligoset 26, oligosets 29 to 31, and oligosets 35 to 36, even if human nucleic acid was added, the detection sensitivity of the nucleic acid amplification reaction was not influenced. In oligoset 27 and oligoset 34, the detection sensitivity of the nucleic acid amplification reaction decreased by about 10 times by addition of human nucleic acid. In oligoset 25, the detection sensitivity of the nucleic acid amplification reaction decreased by about 100 times by addition of human nucleic acid. In oligoset 28, oligoset 32, and oligoset 33, the detection sensitivity of the nucleic acid amplification reaction decreased by about 1000 times or more by addition of human nucleic acid.

### Example 2

### [Preparation of test sample]

A target microorganism was added, at 10 CFU/mL and 100 CFU/mL, to a suspension of 1×10⁶ cells/mL of Jurkat cells derived from human leukemic T-cells as human-derived cells to prepare 1 mL of test samples. As target microorganisms, bacterial strains shown in Table 15 were used.

### [Pretreatment of test sample]

Subsequently, extracellularly present free nucleic acid was removed by enzymatic treatment by TurboNuclease, the enzyme was deactivated, and the cells were then crushed using Easy Beads (AMR, Inc.) to obtain a cell lysate solution. Subsequently, nucleic acid was extracted from the cell lysate solution using QIAamp UCP DNA Micro Kit (QIAGEN N.V.) to obtain 40 µL of a nucleic acid extract.

### [Test method]

The reaction solution to be used in the real-time PCR method was prepared as shown in Table 16. The combinations of forward primers, probes, and reverse primers (all are manufactured by NIHON TECHNO SERVICE CO., LTD.) used in 10× Primer/Probe Mix are shown in Table 15. Regarding the probes, FAM is attached to the 5' end, and BHQ1 is attached to the 3' end. The real-time PCR was performed using CFX Connect (Bio-Rad Laboratories, Inc.) under a reaction condition of heating at 95°C for 10 seconds and then 45 cycles of heating at 95°C for 10 seconds and at 66°C for 60 seconds (Table 17). The test was performed twice.

**[Table 15]**

| | Bacterial species | Bacterial strain number | Oligoset |
|---|---|---|---|
| Bacterium | *Bacillus subtilis* | ATCC 6633 | 20 |
| | *Clostridium sporogenes* | ATCC 11437 | 20 |
| | *Pseudomonas aeruginosa* | ATCC 9027 | 20 |
| | *Staphylococcus aureus* | ATCC 6538 | 20 |
| Fungus | *Aspergillus brasiliensis* | NCPF 2275 | 21 |
| | *Candida albicans* | ATCC 10231 | 21 |

**[Table 16]**

| Reagent | Volume |
|---|---|
| Platinum^{™} Taq DNA Polymerase, DNA-free | 0.25 µL |
| 10X PCR Buffer (-MgCl₂) | 2.5 µL |
| 50 mM MgCl₂ | 0.75 µL |
| dNTP Mix (10 mM each) | 0.5 µL |
| 10xPrimer/Probe Mix | 2.5 µL |
| Nucleic acid extract | 10 µL |
| Water | 8.5 µL |
| Total | 25 µL |

**[Table 17]**

| Temperature | Time | Note |
|---|---|---|
| 95°C | 10 sec | 1 cycle |
| 5°C | 10 sec | 45 cycles |
| 66°C | 60 sec | |

### [Results]

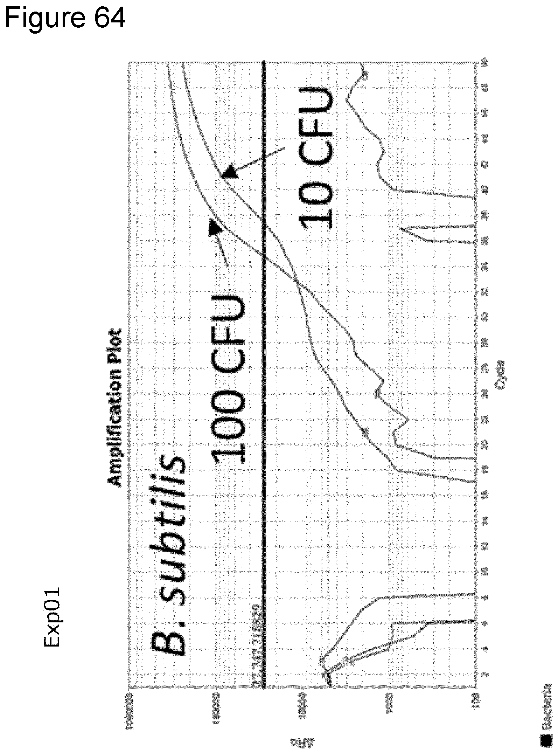

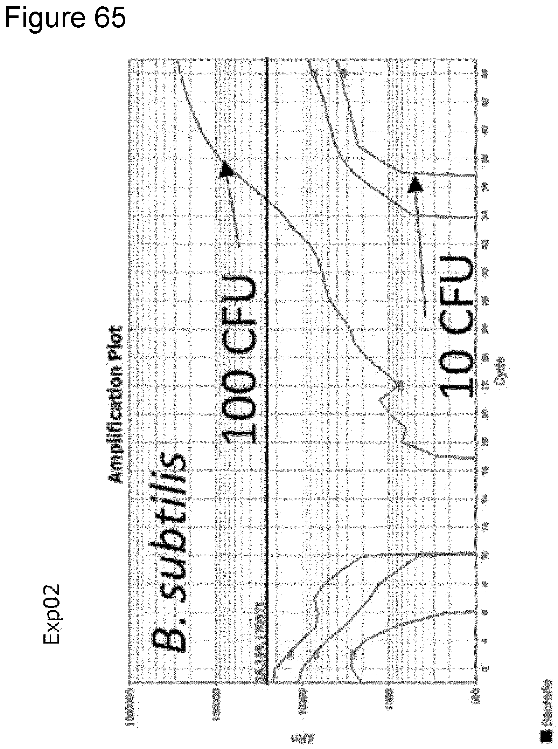

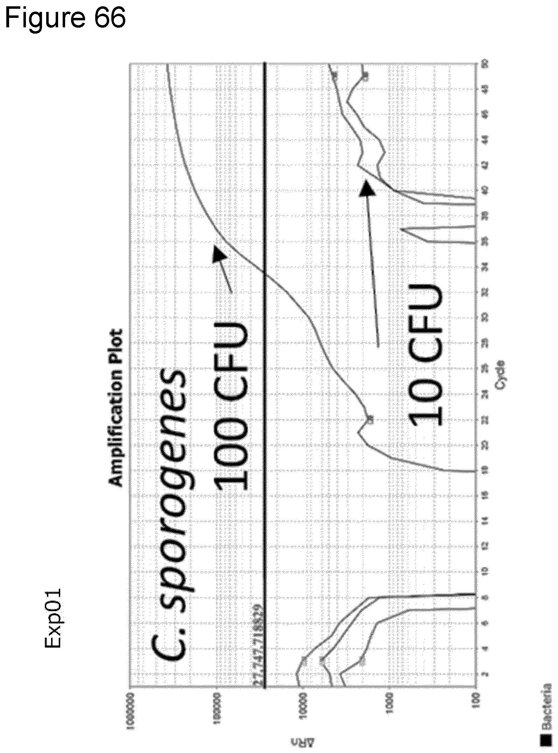

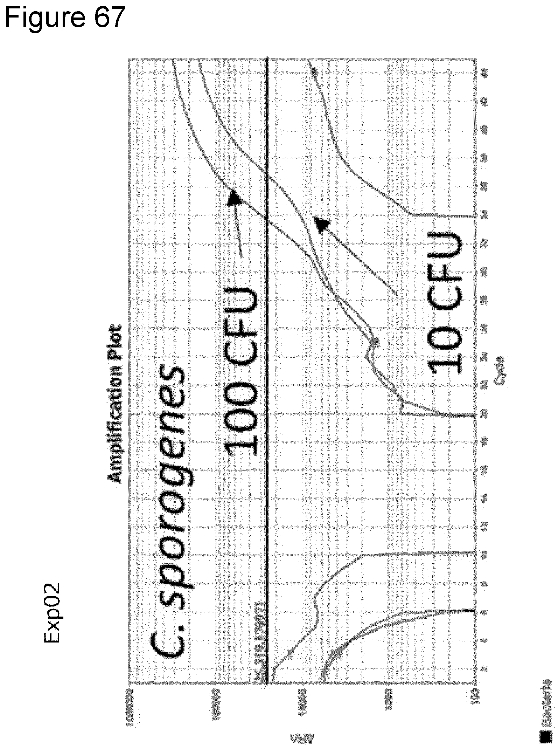

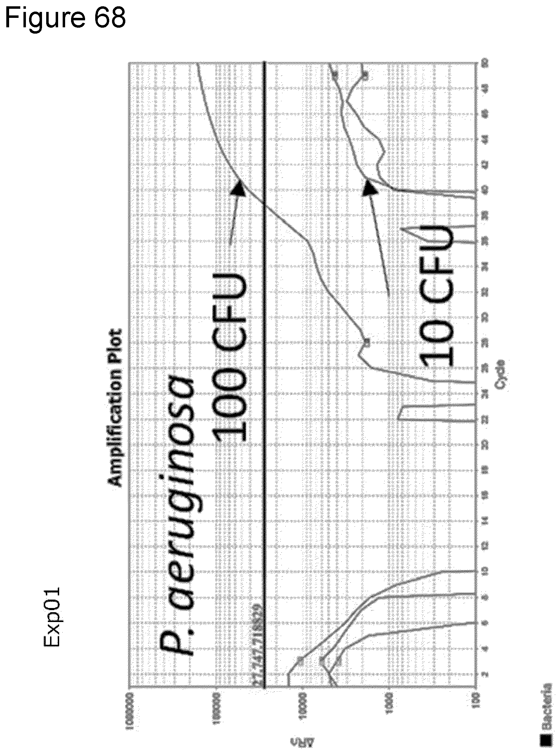

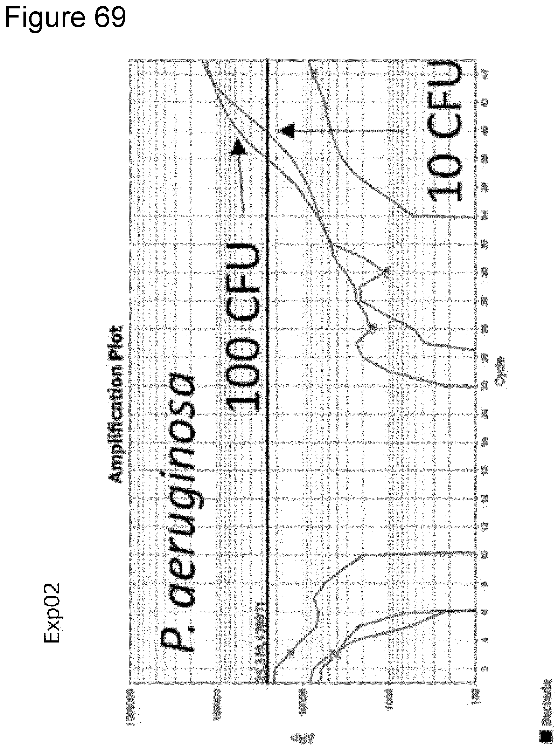

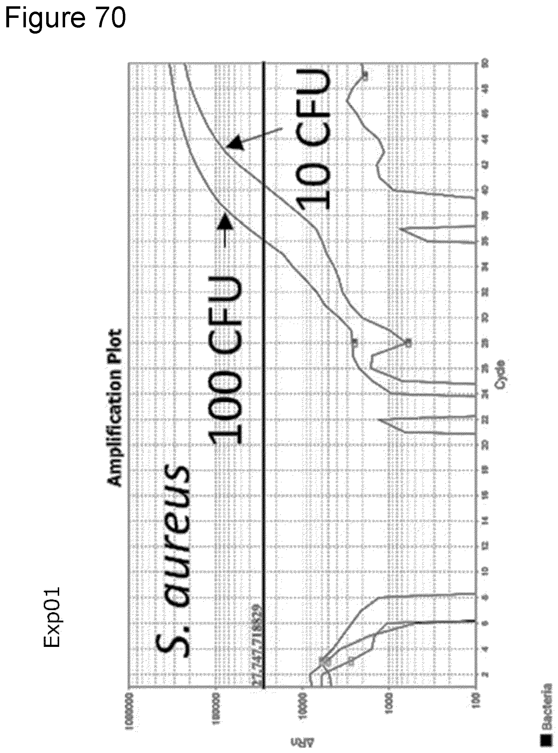

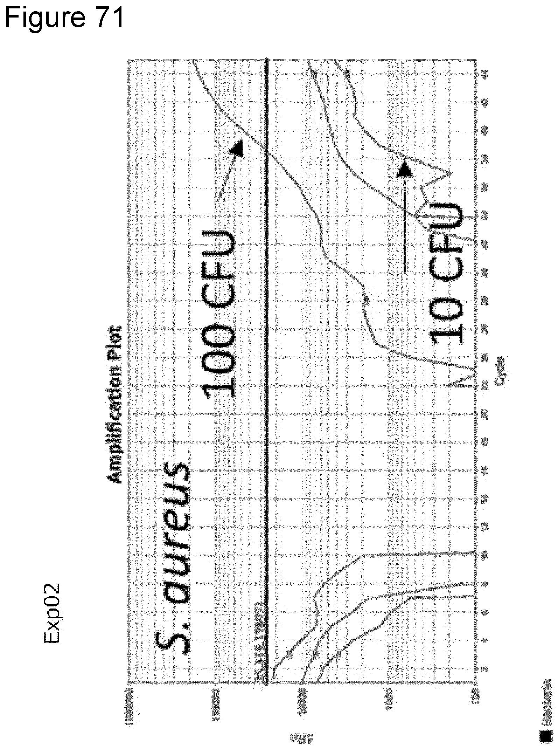

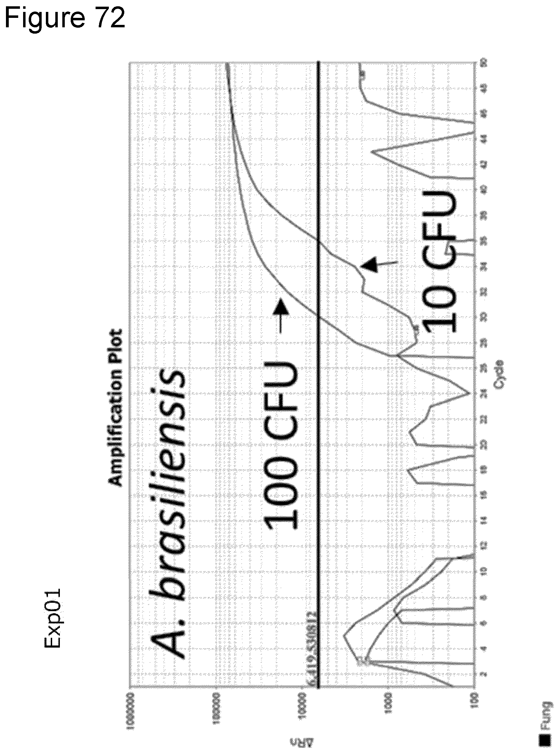

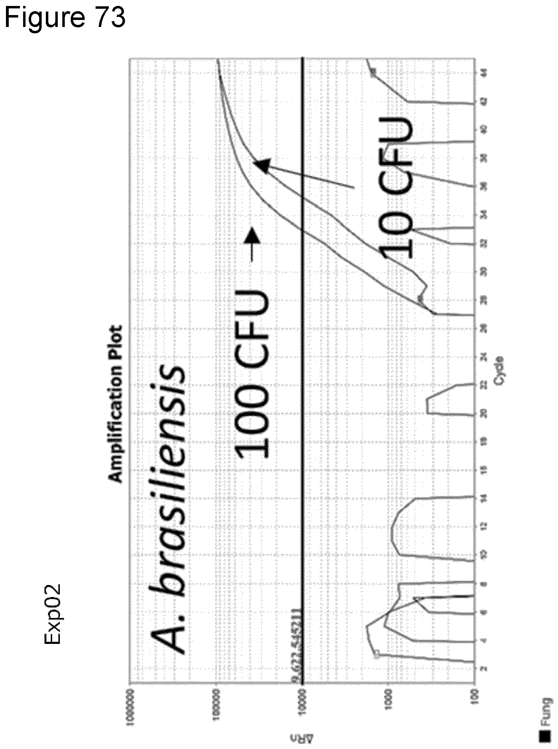

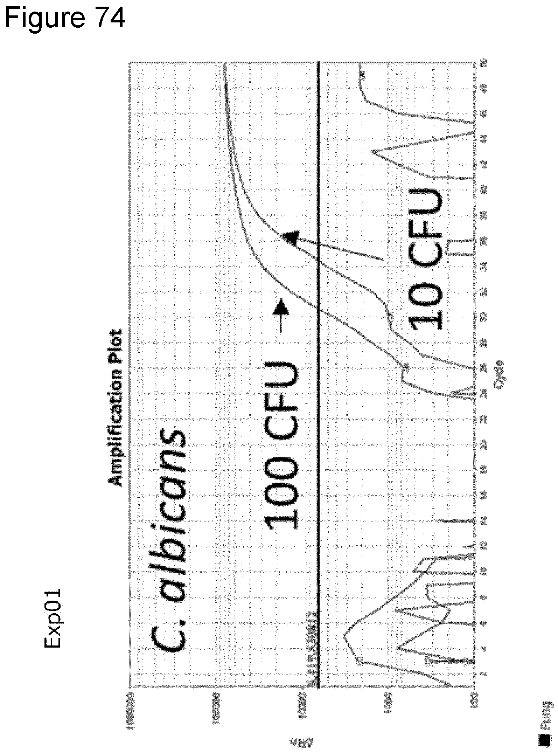

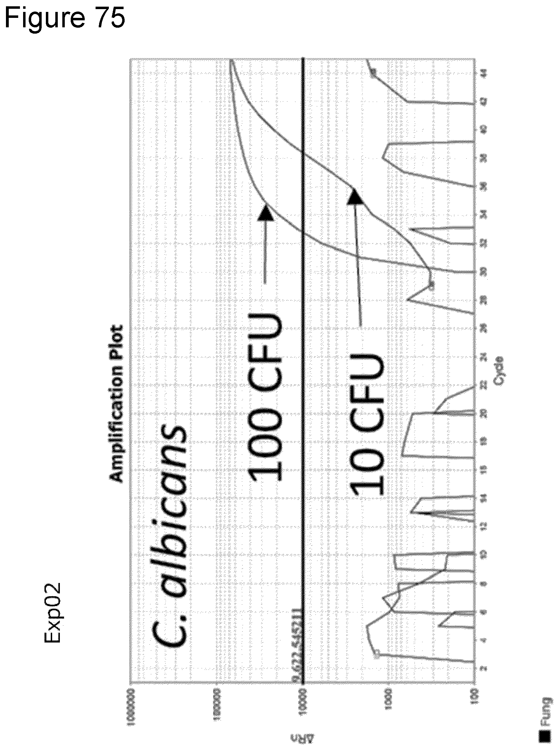

Amplification curves using bacteria are shown in Figures 64 to 71, amplification curves using fungi are shown in Figures 72 to 75, and possibility of sterility tests using bacteria and fungi is shown in Table 18.

In this test, since 10 µL of 40 µL of the nucleic acid extract was subjected to real-time PCR, the amounts of human-derived cells and target microorganism included in the test sample are one-fourth of those in the test sample. In Table 18, "Detected" indicated that the presence of a target microorganism could be detected by the rapid sterility test in the presence of human-derived cells, and "Undetected" indicated that the presence of a target microorganism could not be detected by the rapid sterility test. The results were indicated as "2/2" when two of two tests could detect the presence of a target microorganism and as "1/2" when one of two tests could detect the presence of a target microorganism.

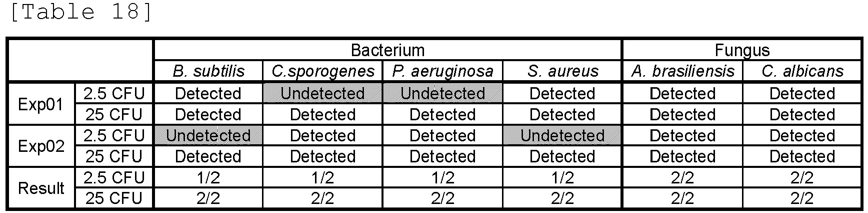

### Possibility of sterility test of bacteria (Table 18)

In oligoset 20, 25 CFU of a target microorganism included in a test sample added with human-derived cells could be detected in two of two tests. Furthermore, in oligoset 20, 2.5 CFU of a target microorganism included in a test sample added with human-derived cells could be detected in one of two tests. Here, the reason why the microorganism could not be detected is probably because the amount of the target microorganism in PCR was 2.5 CFU/test, very small.

From the above, it was demonstrated that even if a test sample added with human-derived cells is used, oligoset 20 can be used in rapid sterility tests.

### Possibility of sterility test of fungi (Table 18)

In oligoset 21, 25 CFU and 2.5 CFU of a target microorganism included in a test sample added with human-derived cells could be detected in two of two tests. From the above, it was demonstrated that even if a test sample added with human-derived cells is used, oligoset 21 can be used in rapid sterility tests.

### Industrial Applicability

It is possible to reduce inhibition of the nucleic acid amplification reaction of target nucleic acid due to human nucleic acid in a nucleic acid amplification method and to detect comprehensively bacterial or fungal nucleic acid by using the present invention, and the present invention can be used in rapid sterility tests in regenerative medicine.

## Claims

1. A rapid sterility test method for detecting presence of bacterial nucleic acid included in a test sample including human nucleic acid by a nucleic acid amplification method, wherein an oligoset that is used in the nucleic acid amplification method comprises:
(1) one or more probes selected from the group consisting of a probe group PM1 of from 12 to 42 nucleotides, including a nucleotide sequence set forth in SEQ ID NO: 18 or a nucleotide sequence having insertion, deletion, or substitution of from 1 to 3 nucleotides in this nucleotide sequence, a probe group PM2 of from 8 to 42 nucleotides, including a nucleotide sequence set forth in SEQ ID NO: 27 or a nucleotide sequence having insertion, deletion, or substitution of from 1 to 3 nucleotides in this nucleotide sequence, and a probe group PM3 of from 7 to 42 nucleotides, including a nucleotide sequence set forth in SEQ ID NO: 30 or a nucleotide sequence having insertion, deletion, or substitution of from 1 to 3 nucleotides in this nucleotide sequence; and
(2) one or more reverse primers in a primer group RM of from 8 to 30 nucleotides, including a nucleotide sequence set forth in SEQ ID NO: 4 or a nucleotide sequence having insertion, deletion, or substitution of from 1 to 3 nucleotides in this nucleotide sequence.

2. The rapid sterility test method according to claim 1, wherein
the probes are one or more probes selected from the group consisting of nucleotide sequences set forth in SEQ ID NOs: 21 to 26, SEQ ID NO: 29, and SEQ ID NO: 32 or nucleotide sequences having insertion, deletion, or substitution of from 1 to 3 nucleotides in each of these nucleotide sequences; and
the reverse primers are one or more reverse primers selected from the group consisting of nucleotide sequences set forth in SEQ ID NOs: 7 to 17 or nucleotide sequences having insertion, deletion, or substitution of from 1 to 3 nucleotides in each of these nucleotide sequences.

3. The rapid sterility test method according to claim 1 or 2, wherein the oligoset further comprises (3) one or more forward primers in a primer group FM of from 13 to 50 nucleotides, including a nucleotide sequence set forth in SEQ ID NO: 1 or a nucleotide sequence having insertion, deletion, or substitution of from 1 to 3 nucleotides in this nucleotide sequence.

4. The rapid sterility test method according to claim 3, wherein the forward primers are one or more forward primers selected from the group consisting of a nucleotide sequence set forth in SEQ ID NO: 3 or a nucleotide sequence having insertion, deletion, or substitution of from 1 to 3 nucleotides in this nucleotide sequence.

5. The rapid sterility test method according to any one of claims 1 to 4, wherein the test sample includes 1 to 1 × 10⁵ ng of the human nucleic acid.

6. The rapid sterility test method according to any one of claims 1 to 5, wherein the number of copies of the bacterial nucleic acid to be detected is 1 to 100 copies.

7. The rapid sterility test method according to any one of claims 1 to 6, wherein a nucleic acid extract is used, which is nucleic acid included in a human-derived cell and/or bacterium and extracted from an enzyme-treated specimen prepared by adding a nuclease to a test sample at least including the human-derived cell as the human nucleic acid and the bacterium as the bacterial nucleic acid and removing and/or deactivating the nuclease.

8. The rapid sterility test method according to claim 7, wherein the number of cells to be detected is 1 to 100 CFU.

9. A rapid sterility test method for detecting presence of fungal nucleic acid included in a test sample including human nucleic acid by a nucleic acid amplification method, wherein an oligoset that is used in the nucleic acid amplification method comprises:
(1) one or more forward primers in a primer group FY1 of from 11 to 44 nucleotides, including a nucleotide sequence set forth in SEQ ID NO: 33 or a nucleotide sequence having insertion, deletion, or substitution of from 1 to 3 nucleotides in this nucleotide sequence, and a primer group FY2 of from 16 to 44 nucleotides, including a nucleotide sequence set forth in SEQ ID NO: 45 or a nucleotide sequence having insertion, deletion, or substitution of from 1 to 3 nucleotides in this nucleotide sequence; and
(2) one or more reverse primers in a primer group RY of from 17 to 44 nucleotides, including a nucleotide sequence set forth in SEQ ID NO: 51 or a nucleotide sequence having insertion, deletion, or substitution of from 1 to 3 nucleotides in this nucleotide sequence.

10. The rapid sterility test method according to claim 9, wherein
the forward primers are one or more forward primers selected from the group consisting of nucleotide sequences set forth in SEQ ID NOs: 37 to 44 or nucleotide sequences set forth in SEQ ID NOs: 46 to 48 or nucleotide sequences having insertion, deletion, or substitution of from 1 to 3 nucleotides in each of these nucleotide sequences; and
the reverse primers are one or more reverse primers selected from the group consisting of nucleotide sequences set forth in SEQ ID NOs: 52 to 55 or nucleotide sequences having insertion, deletion, or substitution of from 1 to 3 nucleotides in each of these nucleotide sequences.

11. The rapid sterility test method according to claim 9 or 10, wherein the oligoset further comprises (3) one or more probes in a probe group PY of from 17 to 50 nucleotides, including a nucleotide sequence set forth in SEQ ID NO: 56 or a nucleotide sequence having insertion, deletion, or substitution of from 1 to 3 nucleotides in this nucleotide sequence.

12. The rapid sterility test method according to claim 11, wherein the probes are one or more probes selected from the group consisting of a nucleotide sequence set forth in SEQ ID NO: 58 or a nucleotide sequence having insertion, deletion, or substitution of from 1 to 3 nucleotides in this nucleotide sequence.

13. The rapid sterility test method according to any one of claims 9 to 12, wherein the test sample includes 1 to 1 × 10⁵ ng of the human nucleic acid.

14. The rapid sterility test method according to any one of claims 9 to 13, wherein the number of copies of the fungal nucleic acid to be detected is 1 to 100 copies.

15. The rapid sterility test method according to any one of claims 9 to 14, wherein a nucleic acid extract is used, which is nucleic acid included in a human-derived cell and/or fungus and extracted from an enzyme-treated specimen prepared by adding a nuclease to a test sample at least including the human-derived cell as the human nucleic acid and the fungus as the fungal nucleic acid and removing and/or deactivating the nuclease.

16. The rapid sterility test method according to claim 15, wherein the number of fungi to be detected is 1 to 100 CFU.

17. The rapid sterility test method according to any one of claims 7, 8, 15, and 16, wherein the human-derived cell is in 1 to 1 × 10⁷ cells.

18. The rapid sterility test method according to any one of claims 1 to 17, wherein the nucleic acid amplification method is a PCR method or a real-time PCR method.

19. A real-time PCR kit for use in the rapid sterility test method according to any one of claims 1 to 18.
